# EUROPEAN PATENT SPECIFICATION

(11) **EP 4 346 795 B1**
(45) Date of publication and mention of the grant of the patent: **05.03.2025**
(21) Application number: 22740572.7
(22) Date of filing: 01.06.2022
(51) Int. Cl.: A61K 31/20, A61K 31/55, A61P 13/12

(54) **COMPOSITIONS CONTAINING BEMPEDOIC ACID ALONE OR IN COMBINATION WITH TOLVAPTAN FOR USE IN A METHOD FOR TREATING AUTOSOMAL DOMINANT POLYCYSTIC KIDNEY DISEASE (ADPKD)**
ZUBEREITUNGEN ENTHALTEND BEMPEDOSÄURE ALLEIN ODER IN KOMBINATION MIT TOLVAPTAN ZUR VERWENDUNG IN EINEM VERFAHREN ZUR BEHANDLUNG VON AUTOSOMAL DOMINANTER POLYZYSTISCHER NIERENERKRANKUNG (ADPKD)
COMPOSITIONS CONTENANT DE L'ACIDE BÉMIPÉDOÏQUE SEUL OU EN COMBINAISON AVEC DU TOLVAPTAN POUR UNE UTILISATION DANS UN PROCÉDÉ DE TRAITEMENT DE LA MALADIE RÉNALE POLYKYSTIQUE DOMINANTE AUTOSOMALE (ADPSKD)

(30) Priority: 02.06.2021 US 202163195843 P; 03.11.2021 US 202163275077 P
(43) Date of publication of application: 10.04.2024
(73) Proprietor: Esperion Therapeutics, Inc., Ann Arbor, MI 48108 (US)
(72) Inventor: PASTOR-SOLER, Nuria M., Altadena, CA 91001 (US); HALLOWS, Kenneth R., Altadena, CA 91001 (US); PINKOSKY, Stephen L., Plymouth, MI 48170 (US); SASIELA, William J., Bloomingdale, NJ 07403 (US); HALL, Ashley F., Camarillo, CA 93010 (US)
(74) Representative: Mewburn Ellis LLP
(86) International application number: PCT/US2022/031729
(87) International publication number: WO 2022/256378

(56) References cited:
- WO-A1-2019/161307
- SANS-ATXER LAIA ET AL: "Tolvaptan in the treatment of autosomal dominant polycystic kidney disease: patient selection and special considerations", INTERNATIONAL JOURNAL OF NEPHROLOGY AND RENOVASCULAR DISEASE, vol. Volume 11, 1 January 2018 (2018-01-01), pages 41 - 51, XP055956391, Retrieved from the Internet <URL:https://www.dovepress.com/getfile.php?fileID=40353> DOI: 10.2147/IJNRD.S125942
- BAYS HAROLD E ET AL: "Bempedoic acid safety analysis: Pooled data from four phase 3 clinical trials", JOURNAL OF CLINICAL LIPIDOLOGY, ELSEVIER, NEW YORK, NY, US, vol. 14, no. 5, 1 September 2020 (2020-09-01), pages 649, XP086329502, ISSN: 1933-2874, [retrieved on 20200902], DOI: 10.1016/J.JACL.2020.08.009
- DATABASE EMBASE [online] ELSEVIER SCIENCE PUBLISHERS, AMSTERDAM, NL; 2021, HALLOWS K R ET AL: "Beneficial effects of bempedoic acid treatment in ADPKD mice", XP002807422, Database accession no. EMB-636330968
- JOURNAL OF THE AMERICAN SOCIETY OF NEPHROLOGY 2021 AMERICAN SOCIETY OF NEPHROLOGY NLD, vol. 32, 2021, pages 403 CONF 20211102 to 20211107 San Diego, CA - Kidney We, ISSN: 1533-3450

## Description

### CROSS-REFERENCE TO RELATED APPLICATIONS

This application claims the benefit of, and priority to, U.S. Patent Application No. 63/195,843 filed June 2, 2021, and U.S. Patent Application No. 63/275,077 filed November 3, 2021.

### BACKGROUND

Autosomal dominant polycystic kidney disease (ADPKD) is a serious genetic disorder characterized by growth of cysts in the kidneys and subsequent loss of kidney function. Worldwide, approximately 4 to 7 million individuals are affected by ADPKD, representing a serious health concern (Akoh; Current management of autosomal dominant polycystic kidney disease; World J. Nephrol.; 2015; 4(4); 468-79). It is the most common genetic cause of end-stage kidney disease, with a prevalence of 1:500 to 1:1000 and 600,000 patients in the U.S. alone (Gabow; Autosomal dominant polycystic kidney disease; The New England Journal of Medicine; 1993; 329; 332-342). Progression of the disease is characterized by enlarging cystic lesions in the kidney and often in the liver and leads to a progressive decline in kidney function, with an associated increase in morbidity and mortality of ADPKD patients (Gabow). Most ADPKD patients have loss-of-function mutations in the polarity protein polycystin-1 (PKD1), and the therapeutic options are limited (Torres et al.; Tolvaptan in patients with autosomal dominant polycystic kidney disease; N. Engl. J. Med.; 2012; 367; 2407-2418). As such, the development of new treatment options for ADPKD patients represents an important area of study.

Tolvaptan is the only drug approved by the FDA for treatment of hyponatremia and ADPKD. It functions in the kidneys as an antagonist of vasopressin receptor 2 and causes aquaresis (e.g., excretion of water without significant loss of electrolytes) (Torres). Although tolvaptan is effective at ameliorating the symptoms and slowing the progression of ADPKD, it has a non-ideal safety profile. Specifically, tolvaptan therapy has the dose-dependent side effect of risk of hepatotoxicity, which includes acute liver failure requiring transplantation and fatal liver injury, and therefore requires monthly testing of liver function (Wu et al.; Mechanisms of tolvaptan-induced toxicity in HepG2 cells; Biochem. Pharmacol.; 2015; 95; 324-336). The status of tolvaptan as the only FDA-approved drug for the treatment of ADPKD indicates a need for new therapeutics to treat this condition.

Despite the success of tolvaptan, there remains an unmet need for safe and effective treatment options for patients with ADPKD.

### SUMMARY

The present disclosure provides combination drug therapies comprising an ATP citrate lyase (ACL) inhibitor (e.g., bempedoic acid) and a vasopressin 2 receptor antagonist (e.g., tolvaptan). The present disclosure also provides methods of using the combination drug therapies described herein, including, for example, for the treatment of autosomal dominant polycystic kidney disease (ADPKD) in a subject in need thereof. The disclosure additionally provides methods of using bempedoic acid as a single agent for the treatment of conditions including ADPKD.

In one aspect, the disclosure provides a fixed-dose combination comprising bempedoic acid and tolvaptan.

In various embodiments of the disclosure, the fixed-dose combination comprises about 30 mg to about 240 mg bempedoic acid. In various embodiments of the disclosure, the fixed-dose combination comprises about 5 mg to about 120 mg tolvaptan; or about 5 mg to about 90 mg tolvaptan.

In some embodiments, the disclosure provides a fixed-dose combination comprising about 30 mg to about 240 mg bempedoic acid and about 5 mg to about 90 mg tolvaptan; and a fixed-dose combination comprising about 30 mg to about 240 mg bempedoic acid and about 5 mg to about 60 mg tolvaptan.

In various embodiments of the disclosure, the fixed-dose combination comprises about 90 mg, about 60 mg, about 45 mg, about 30 mg, about 15 mg, about 10 mg, or about 5 mg tolvaptan. In certain embodiments of the disclosure, the fixed-dose combination comprises about 180 mg bempedoic acid.

In certain embodiments, the disclosure provides a fixed-dose combination comprising about 180 mg bempedoic acid and about 5 mg tolvaptan; a fixed-dose combination comprising about 180 mg bempedoic acid and about 10 mg tolvaptan; a fixed-dose combination comprising about 180 mg bempedoic acid and about 15 mg tolvaptan; a fixed-dose combination comprising about 180 mg bempedoic acid and about 30 mg tolvaptan; a fixed-dose combination comprising about 180 mg bempedoic acid and about 45 mg tolvaptan; a fixed-dose combination comprising about 180 mg bempedoic acid and about 60 mg tolvaptan; and a fixed-dose combination comprising about 180 mg bempedoic acid and about 90 mg tolvaptan.

In various embodiments of the disclosure, the fixed-dose combination is formulated for oral delivery. In certain embodiments of the disclosure, the fixed-dose combination is formulated as an oral solid dosage form selected from the group consisting of a tablet, a capsule, a softgel capsule, a pill, a solution, and a suspension.

In another aspect, the disclosure provides a pharmaceutical composition comprising bempedoic acid, tolvaptan, and one or more pharmaceutically acceptable excipients.

In various embodiments of the disclosure, the pharmaceutical composition comprises about 30 mg to about 240 mg bempedoic acid. In various embodiments, the pharmaceutical composition comprises about 5 mg to about 120 mg tolvaptan; or about 5 mg to about 90 mg tolvaptan.

In some embodiments, the disclosure provides a pharmaceutical composition comprising about 30 mg to about 240 mg bempedoic acid, about 5 mg to about 90 mg tolvaptan, and one or more pharmaceutically acceptable excipients; and a pharmaceutical composition comprising about 30 mg to about 240 mg bempedoic acid, about 5 mg to about 60 mg tolvaptan, and one or more pharmaceutically acceptable excipients.

In various embodiments of the disclosure, the pharmaceutical composition comprises about 90 mg, about 60 mg, about 45 mg, about 30 mg, about 15 mg, about 10 mg, or about 5 mg tolvaptan. In certain embodiments of the disclosure, the pharmaceutical composition comprises about 180 mg bempedoic acid.

In particular embodiments, the disclosure provides a pharmaceutical composition comprising about 180 mg bempedoic acid, about 5 mg tolvaptan, and one or more pharmaceutically acceptable excipients; a pharmaceutical composition comprising about 180 mg bempedoic acid, about 10 mg tolvaptan, and one or more pharmaceutically acceptable excipients; a pharmaceutical composition comprising about 180 mg bempedoic acid, about 15 mg tolvaptan, and one or more pharmaceutically acceptable excipients; a pharmaceutical composition comprising about 180 mg bempedoic acid, about 30 mg tolvaptan, and one or more pharmaceutically acceptable excipients; a pharmaceutical composition comprising about 180 mg bempedoic acid, about 45 mg tolvaptan, and one or more pharmaceutically acceptable excipients; a pharmaceutical composition comprising about 180 mg bempedoic acid, about 60 mg tolvaptan, and one or more pharmaceutically acceptable excipients; and a pharmaceutical composition comprising about 180 mg bempedoic acid, about 90 mg tolvaptan, and one or more pharmaceutically acceptable excipients.

In various embodiments of the disclosure, the pharmaceutical composition is formulated for oral delivery. In certain embodiments of the disclosure, the pharmaceutical composition is formulated as an oral solid dosage form selected from the group consisting of a tablet, a capsule, a softgel capsule, a pill, a solution, and a suspension.

In certain embodiments of the disclosure, the pharmaceutical composition provides immediate release of bempedoic acid. In other embodiments of the disclosure, the pharmaceutical composition provides sustained release of bempedoic acid.

In another aspect, the disclosure provides a method of treating ADPKD in a subject receiving tolvaptan therapy, the method comprising administering to the subject an effective amount of bempedoic acid. In another aspect, the disclosure provides a method of slowing the progression of ADPKD in a subject receiving tolvaptan therapy, the method comprising administering to the subject an effective amount of bempedoic acid. In another aspect, the disclosure provides a method of preventing kidney failure in a subject with ADPKD receiving tolvaptan therapy, the method comprising administering to the subject an effective amount of bempedoic acid.

In various embodiments of the disclosure, the effective amount of bempedoic acid is about 30 mg to about 240 mg. In certain embodiments of the disclosure, the effective amount of bempedoic acid is about 180 mg.

In various embodiments of the disclosure, the subject receiving the effective amount of bempedoic acid and tolvaptan therapy has a lower yearly decrease in estimated glomerular filtration rate than a subject not receiving the effective amount of bempedoic acid and tolvaptan therapy, or receiving only the effective amount of bempedoic acid, or receiving only tolvaptan therapy. In various embodiments of the disclosure, the subject receiving the effective amount of bempedoic acid and tolvaptan therapy has a lower yearly increase in total kidney volume than a subject not receiving the effective amount of bempedoic acid and tolvaptan therapy, or receiving only the effective amount of bempedoic acid, or receiving only tolvaptan therapy. In various embodiments of the disclosure, the subject receiving the effective amount of bempedoic acid and tolvaptan therapy has a lower composite incidence of clinical endpoints selected from worsening kidney function, kidney pain, hypertension, and albuminuria than a subject not receiving the effective amount of bempedoic acid and tolvaptan therapy, or receiving only the effective amount of bempedoic acid, or receiving only tolvaptan therapy.

In various embodiments of the disclosure, the subject receiving the effective amount of bempedoic acid and tolvaptan therapy has higher liver AMPK activity than a subject not receiving the effective amount of bempedoic acid and tolvaptan therapy, or receiving only tolvaptan therapy. In various embodiments of the disclosure, the subject receiving the effective amount of bempedoic acid and tolvaptan therapy has a higher degree of liver acetyl-coenzyme A carboxylase (ACC) phosphorylation than a subject not receiving the effective amount of bempedoic acid and tolvaptan therapy, or receiving only tolvaptan therapy. In various embodiments of the disclosure, the subject receiving the effective amount of bempedoic acid and tolvaptan therapy has a higher degree of peroxisome proliferator-activated receptor gamma coactivator 1-α (PGC1α) activity than a subject receiving only tolvaptan therapy.

In various embodiments of the disclosure, the effective amount of bempedoic acid is delivered orally.

In another aspect, the disclosure provides a method of treating ADPKD in a subject in need thereof, the method comprising administering to the subject a fixed-dose combination of bempedoic acid and tolvaptan, wherein the fixed-dose combination comprises about 30 mg to about 240 mg bempedoic acid and about 5 mg to about 90 mg tolvaptan. In another aspect, the disclosure provides a method of slowing the progression of ADPKD in a subject in need thereof, the method comprising administering to the subject a fixed-dose combination of bempedoic acid and tolvaptan, wherein the fixed-dose combination comprises about 30 mg to about 240 mg bempedoic acid and about 5 mg to about 90 mg tolvaptan. In another aspect, the disclosure provides a method of preventing kidney failure in a subject with ADPKD, the method comprising administering to the subject a fixed-dose combination of bempedoic acid and tolvaptan, wherein the fixed-dose combination comprises about 30 mg to about 240 mg bempedoic acid and about 5 mg to about 90 mg tolvaptan.

In various embodiments of the disclosure, the subject receiving the fixed-dose combination has a lower yearly decrease in estimated glomerular filtration rate than a subject not receiving the fixed-dose combination, or receiving only about 30 mg to about 240 mg bempedoic acid, or receiving only about 5 mg to about 90 mg tolvaptan. In various embodiments of the disclosure, the subject receiving the fixed-dose combination has a lower yearly increase in total kidney volume than a subject not receiving the fixed-dose combination, or receiving only about 30 mg to about 240 mg bempedoic acid, or receiving only about 5 mg to about 90 mg tolvaptan. In various embodiments of the disclosure, the subject receiving the fixed-dose combination has a lower composite incidence of clinical endpoints selected from worsening kidney function, kidney pain, hypertension, and albuminuria than a subject not receiving the fixed-dose combination, or receiving only about 30 mg to about 240 mg bempedoic acid, or receiving only about 5 mg to about 90 mg tolvaptan.

In various embodiments of the disclosure, the subject receiving the fixed-dose combination has higher liver AMPK activity than a subject not receiving the fixed-dose combination, or receiving only about 5 mg to about 90 mg tolvaptan. In various embodiments of the disclosure, the subject receiving the fixed-dose combination has a higher degree of liver ACC phosphorylation than a subject not receiving the fixed-dose combination, or receiving only about 5 mg to about 90 mg tolvaptan. In various embodiments of the disclosure, the subject receiving the fixed-dose combination has a higher degree of peroxisome proliferator-activated receptor gamma coactivator 1-α (PGC1α) activity than a subject receiving only about 5 mg to about 90 mg tolvaptan.

In various embodiments of the disclosure, the fixed-dose combination is delivered orally.

In various embodiments of the disclosure, the fixed-dose combination comprises about 180 mg bempedoic acid. In certain embodiment of the disclosure, the fixed-dose combination comprises about 5 mg, about 10 mg, about 15 mg, about 30 mg, about 45 mg, about 60 mg, or about 90 mg tolvaptan.

In another aspect, the disclosure provides a method of treating ADPKD in a subject in need thereof, the method comprising administering to the subject an effective amount of bempedoic acid and tolvaptan. In another aspect, the disclosure provides a method of slowing the progression of ADPKD in a subject in need thereof, the method comprising administering to the subject an effective amount of bempedoic acid and tolvaptan. In another aspect, the disclosure provides a method of preventing kidney failure in a subject with ADPKD in need thereof, the method comprising administering to the subject an effective amount of bempedoic acid and tolvaptan.

In various embodiments of the disclosure, the effective amount of bempedoic acid and tolvaptan comprises about 30 mg to about 240 mg bempedoic acid. In various embodiments of the disclosure, the effective amount of bempedoic acid and tolvaptan comprises about 180 mg bempedoic acid. In various embodiments of the disclosure, the effective amount of bempedoic acid and tolvaptan comprises about 5 mg, about 10 mg, about 15 mg, about 30 mg, about 45 mg, about 60 mg, or about 90 mg tolvaptan.

In various embodiments of the disclosure, the subject receiving the effective amount of bempedoic acid and tolvaptan has a lower yearly decrease in estimated glomerular filtration rate than a subject receiving a placebo, or receiving only bempedoic acid, or receiving only tolvaptan. In various embodiments of the disclosure, the subject receiving the effective amount of bempedoic acid and tolvaptan has a lower yearly increase in total kidney volume than a subject receiving a placebo, or receiving only bempedoic acid, or receiving only tolvaptan. In various embodiments of the disclosure, the subject receiving the effective amount of bempedoic acid and tolvaptan has a lower composite incidence of clinical endpoints selected from worsening kidney function, kidney pain, hypertension, and albuminuria than a subject receiving a placebo, or receiving only bempedoic acid, or receiving only tolvaptan.

In various embodiments of the disclosure, the subject receiving the effective amount of bempedoic acid and tolvaptan has higher liver AMPK activity than a subject receiving a placebo, or receiving only tolvaptan. In various embodiments of the disclosure, the subject receiving the effective amount of bempedoic acid and tolvaptan has a higher degree of liver ACC phosphorylation than a subject receiving a placebo, or receiving only tolvaptan. In various embodiments of the disclosure, the subject receiving the effective amount of bempedoic acid and tolvaptan has a higher degree of peroxisome proliferator-activated receptor gamma coactivator 1-α (PGC1α) activity than a subject receiving only tolvaptan.

In another aspect, the disclosure provides a method of treating ADPKD in a subject in need thereof, the method comprising administering to the subject an effective amount of bempedoic acid. In another aspect, the disclosure provides a method of slowing the progression of ADPKD in a subject in need thereof, the method comprising administering to the subject an effective amount of bempedoic acid. In another aspect, the disclosure provides a method of preventing kidney failure in a subject with ADPKD, the method comprising administering to the subject an effective amount of bempedoic acid.

In various embodiments of the disclosure, the effective amount bempedoic acid is about 30 mg to about 240 mg. In certain embodiments of the disclosure, the effective amount of bempedoic acid is about 180 mg.

In various embodiments of the disclosure, the subject receiving the effective amount of bempedoic acid has a lower yearly decrease in estimated glomerular filtration rate than a subject receiving a placebo, or has an equivalent or a lower yearly decrease in estimated glomerular filtration rate than a subject receiving tolvaptan therapy. In various embodiments, the subject receiving the effective amount of bempedoic acid has a lower yearly increase in total kidney volume than a subject receiving a placebo, or has an equivalent or a lower yearly increase in total kidney volume than a subject receiving tolvaptan therapy. In various embodiments of the disclosure, the subject receiving the effective amount of bempedoic acid has a lower composite incidence of clinical endpoints selected from worsening kidney function, kidney pain, hypertension, and albuminuria than a subject receiving a placebo, or has an equivalent or a lower composite incidence of clinical endpoints selected from worsening kidney function, kidney pain, hypertension, and albuminuria than a subject receiving tolvaptan therapy. In various embodiments of the disclosure, the effective amount of bempedoic acid is delivered orally.

In various embodiments of the disclosure, the subject is a human.

### BRIEF DESCRIPTION OF FIGURES

**FIG. 1A** is an immunoblot assay showing the fatty acid transport protein 2 (FATP2, also known as very long-chain acyl-CoA synthetase (ACSVL1) and/or the gene SLC27A2) protein expression in homogenates of different mouse tissues (liver, lung, kidney, spleen, and muscle), proximal tubule (PT)-derived kidney cell lines (S3, PKD1 Null), and inner medullary collecting duct (IMCD)-derived kidney cell lines (IMCD3 [WT] and ID1-3E5 [PKD]).
**FIG. 1B** is an immunoblot assay showing the FATP2a 70kDa and FATP2b 55kDa isoform expression in homogenates of wild type and ADPKD mutant (Pkd1RC/RC) mouse kidney tissue.
**FIG. 1C** is an immunoblot assay showing the ATP citrate lyase (ACLY) protein expression and activity in inner medullary collecting duct-derived Pkd1-/- mouse kidney epithelial cells compared to wild type.
**FIG. 1D** is a histogram comparing the ratios of phosphorylated ACLY to total ACLY expression in IMCD-derived wild-type and PKD1 knockout cells.
**FIG.** 1E is an immunoblot assay showing the ACLY protein expression and activity in proximal tubule (PT)-derived Pkd1-heterozygous and Pkd1-null mouse kidney epithelial cells compared to wild type.
**FIG. 1F** is a histogram comparing the ratios of phosphorylated ACLY to total ACLY expression in PT-derived Pkd1-heterozygous and Pkd1-null cells.
**FIG. 2A** shows photographs comparing cyst growth in PT-derived Pkd1-/- cell 3D cultures that are untreated, treated with ETC-1002, or treated with SB-204990, with and without metformin.
**FIG. 2B** is a plot comparing the cystic area of PT-derived Pkd1-/- cell 3D cultures that are untreated, treated with ETC-1002, or treated with SB-204990, with and without metformin.
**FIG. 2C** shows photographs comparing cyst growth in IMCD-derived Pkd1-/- cell 3D cultures that are untreated or treated with ETC-1002, with and without metformin.
**FIG. 2D** is a plot comparing the cyst area of IMCD-derived Pkd1-/- cell 3D cultures that are untreated or treated with ETC-1002, with and without metformin.
**FIG. 3A** shows images of H&E stains of kidney sections from Cre+ mice at postnatal day 22 treated with vehicle, bempedoic acid, or tolvaptan; as well as an image of a kidney section from an uninduced control mouse without PKD.
**FIG. 3B** is a histogram comparing the total kidney weight to body weight percent in an early onset, rapidly developing ADPKD mouse model, with groups treated with vehicle, tolvaptan 30 mg/kg/d, tolvaptan 100 mg/kg/d, bempedoic acid 30 mg/kg/d, tolvaptan 30 mg/kg/d + bempedoic acid 30 mg/kg/d, and tolvaptan 100 mg/kg/d + bempedoic acid 30 mg/kg/d; and in an uninduced, untreated control group without PKD.
**FIG. 3C** is a histogram comparing the blood urea nitrogen (BUN) in an early onset, rapidly developing ADPKD mouse model, with groups treated with vehicle, tolvaptan 30 mg/kg/d, tolvaptan 100 mg/kg/d, bempedoic acid 30 mg/kg/d, tolvaptan 30 mg/kg/d + bempedoic acid 30 mg/kg/d, or tolvaptan 100 mg/kg/d + bempedoic acid 30 mg/kg/d; and in an uninduced, untreated control without PKD.
**FIG. 4A** is an immunoblot assay of kidney lysates from the treatment groups described in FIGS. 3B and 3C comparing the kidney injury molecule-1 (KIM-1) expression relative to total protein expression among the treatment groups.
**FIG. 4B** is a histogram comparing the kidney lysate kidney injury molecule-1 (KIM-1) expression relative to total protein expression among the treatment groups described in **FIGS. 3B** and **3C****.**
**FIG. 5A** is an immunoblot assay of kidney lysates from the treatment groups described in **FIGS. 3B** and **3C** comparing the AMPK-pThr¹⁷² expression relative to total protein expression among the treatment groups.
**FIG. 5B** is a histogram comparing the kidney lysate Thr¹⁷² expression relative to total protein expression among the treatment groups described in **FIGS. 3B** and **3C****.**
**FIG. 6A** is an immunoblot assay of kidney lysates from the treatment groups described in **FIGS. 3B** and **3C** comparing the pP70S6K expression relative to total protein expression among the treatment groups.
**FIG. 6B** is a histogram comparing the kidney lysate pP70S6K expression relative to total protein expression among the treatment groups described in **FIGS. 3B** and **3C****.**
**FIG.** 7A is an immunoblot assay of kidney lysates from the treatment groups described in **FIGS. 3B** and **3C** comparing the pERK expression relative to total protein expression among the treatment groups.
**FIG. 7B** is a histogram comparing the kidney lysate pERK expression relative to total protein expression among the treatment groups described in **FIGS. 3B** and **3C****.**
**FIG. 8A** is an immunoblot assay of kidney lysates from the treatment groups described in **FIGS. 3B** and **3C** comparing the pACLY expression relative to total protein expression among the treatment groups.
**FIG. 8B** is a histogram comparing the kidney lysate pACLY expression relative to total protein expression among the treatment groups described in **FIGS. 3B** and **3C****.**
**FIG. 9A** is an immunoblot assay of kidney lysates from the treatment groups described in **FIGS. 3B** and **3C** comparing the NGAL expression relative to total protein expression among the treatment groups.
**FIG. 9B** is a histogram comparing the kidney lysate NGAL expression relative to total protein expression among the treatment groups described in **FIGS. 3B** and **3C****.**
**FIG. 10A** is an immunoblot assay of liver lysates from the treatment groups described in **FIGS. 3B** and **3C** receiving vehicle, bempedoic acid 30 mg/kg/d, tolvaptan 100 mg/kg/d, or tolvaptan 100 mg/kg/d + bempedoic acid 30 mg/kg/d, comparing the pACLY expression relative to total protein expression among the treatment groups.
**FIG. 10B** is a histogram comparing the liver lysate pACLY expression relative to total protein expression among the treatment groups described in **FIG. 10A****.**
**FIG. 11A** is an immunoblot assay of liver lysates from the treatment groups described in **FIGS. 3B** and **3C** receiving vehicle, bempedoic acid 30 mg/kg/d, tolvaptan 100 mg/kg/d, or tolvaptan 100 mg/kg/d + bempedoic acid 30 mg/kg/d, comparing the FATP2 expression relative to total protein expression among the treatment groups
**FIG. 11B** is a histogram comparing the liver lysate FATP2 expression relative to total protein expression among the treatment groups described in **FIGS. 11A****.**
**FIG. 12A** is an immunoblot assay of liver lysates from the treatment groups described in **FIGS. 3B** and **3C** receiving vehicle, bempedoic acid 30 mg/kg/d, tolvaptan 100 mg/kg/d, or tolvaptan 100 mg/kg/d + bempedoic acid 30 mg/kg/d, comparing the pThr172 expression relative to total protein expression among the treatment groups.
**FIG. 12B** is a histogram comparing the liver lysate pThr172 expression relative to total protein expression among the treatment groups described in **FIG 12A****.**
**FIG. 13A** is an immunoblot assay of liver lysates from the treatment groups described in **FIGS. 3B** and **3C** receiving vehicle, bempedoic acid 30 mg/kg/d, tolvaptan 100 mg/kg/d, or tolvaptan 100 mg/kg/d + bempedoic acid 30 mg/kg/d, comparing the pACC expression relative to total protein expression among the treatment groups.
**FIG. 13B** is a histogram comparing the liver lysate pACC expression relative to total protein expression among the treatment groups described in **FIG 13A****.**

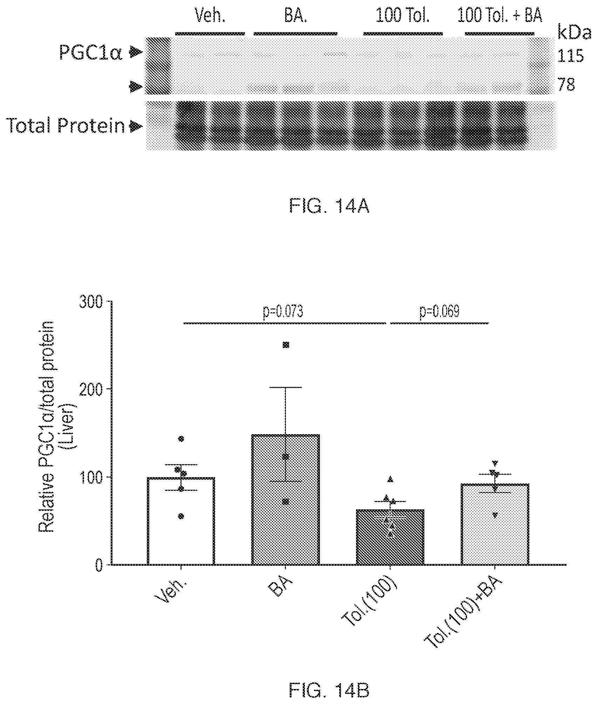

**FIG. 14A** is an immunoblot assay of liver lysates from the treatment groups described in **FIGS. 3B** and **3C** receiving vehicle, bempedoic acid 30 mg/kg/d, tolvaptan 100 mg/kg/d, or tolvaptan 100 mg/kg/d + bempedoic acid 30 mg/kg/d, comparing the PGC1α expression relative to total protein expression among the treatment groups.
**FIG. 14B** is a histogram comparing the liver lysate PGC1α expression relative to total protein expression among the treatment groups described in **FIG. 14A****.**

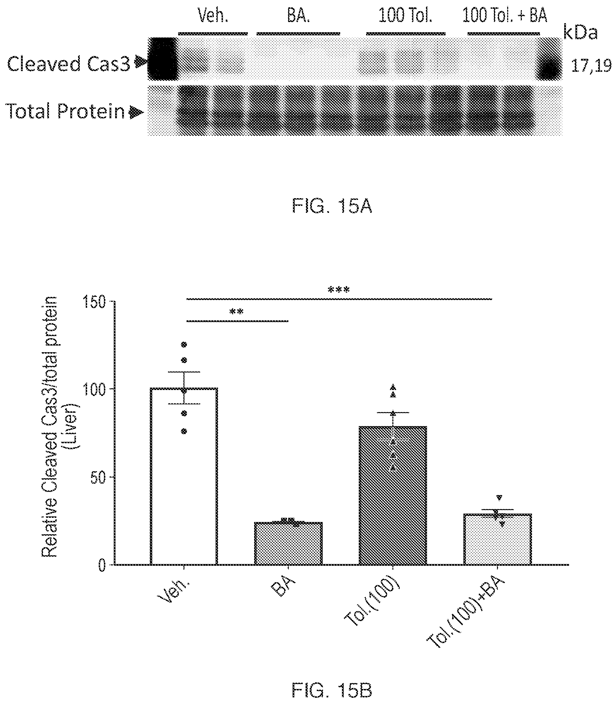

**FIG. 15A** is an immunoblot assay of liver lysates from the treatment groups described in **FIGS. 3B** and **3C** receiving vehicle, bempedoic acid 30 mg/kg/d, tolvaptan 100 mg/kg/d, or tolvaptan 100 mg/kg/d + bempedoic acid 30 mg/kg/d, comparing the cleaved Cas3 expression relative to total protein expression among the treatment groups.
**FIG. 15B** is a histogram comparing the liver lysate cleaved Cas3 expression relative to total protein expression among the treatment groups described in **FIG 15A****.**

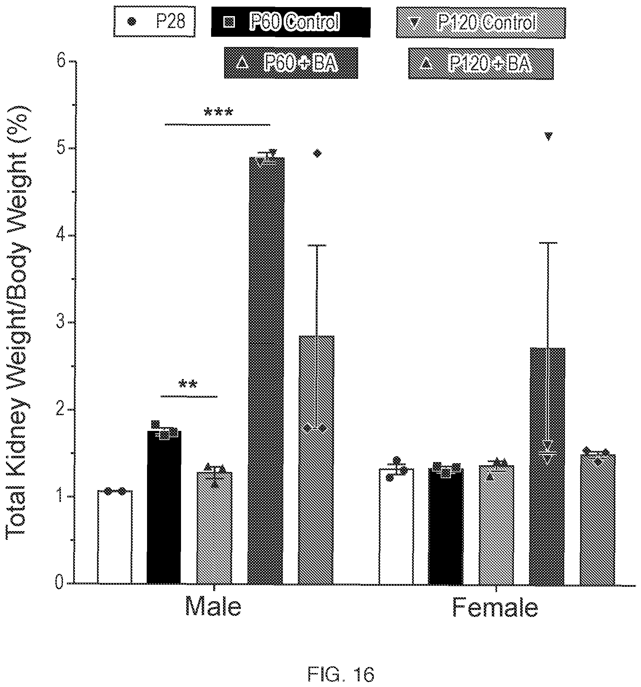

**FIG. 16** is a histogram comparing the total kidney weight to body weight percent in a late onset, slowly developing ADPKD mouse model. Mice were divided into three groups. Two groups were each sacrificed at different points in time after PKD induction, both of which were divided into subgroups receiving either bempedoic acid or control. One group was sacrificed upon induction of PKD to act as a control and facilitate interpretation of the magnitude of treatment effect.

### DETAILED DESCRIPTION

### Definitions

To facilitate an understanding of the present disclosure, a number of terms and phrases are defined below.

Unless defined otherwise, all technical and scientific terms used herein have the same meaning as commonly understood by one of ordinary skill in the art to which this disclosure belongs. The abbreviations used herein have their conventional meaning within the chemical and biological arts. The chemical structures and formulae set forth herein are constructed according to the standard rules of chemical valency known in the chemical arts.

Throughout the description, where compositions and kits are described as having, including, or comprising specific components, or where processes and methods are described as having, including, or comprising specific steps, it is contemplated that, additionally, there are compositions and kits of the present disclosure that consist essentially of, or consist of, the recited components, and that there are processes and methods according to the present disclosure that consist essentially of, or consist of, the recited processing steps.

In the application, where an element or component is said to be included in and/or selected from a list of recited elements or components, it should be understood that the element or component can be any one of the recited elements or components, or the element or component can be selected from a group consisting of two or more of the recited elements or components.

Further, it should be understood that elements and/or features of a composition or a method described herein can be combined in a variety of ways without departing from the spirit and scope of the present disclosure, whether explicit or implicit herein. For example, where reference is made to a particular compound, that compound can be used in various embodiments of compositions of the present disclosure and/or in methods of the present disclosure, unless otherwise understood from the context. In other words, within this application, embodiments have been described and depicted in a way that enables a clear and concise application to be written and drawn, but it is intended and will be appreciated that embodiments may be variously combined or separated without parting from the present teachings and disclosure(s). For example, it will be appreciated that all features described and depicted herein can be applicable to all aspects of the disclosure(s) described and depicted herein.

The articles "a" and "an" are used in this disclosure to refer to one or more than one (i.e., to at least one) of the grammatical object of the article, unless the context is inappropriate. By way of example, "an element" refers to one element or more than one element.

The term "and/or" is used in this disclosure to mean either "and" or "or" unless indicated otherwise.

It should be understood that the expression "at least one of" includes individually each of the recited objects after the expression and the various combinations of two or more of the recited objects unless otherwise understood from the context and use. The expression "and/or" in connection with three or more recited objects should be understood to have the same meaning unless otherwise understood from the context.

The use of the term "include," "includes," "including," "have," "has," "having," "contain," "contains," or "containing," including grammatical equivalents thereof, should be understood generally as open-ended and non-limiting, for example, not excluding additional unrecited elements or steps, unless otherwise specifically stated or understood from the context.

Where the use of the term "about" is before a quantitative value, the present disclosure also includes the specific quantitative value itself, unless specifically stated otherwise. As used herein, the term "about" refers to a ±10% variation from the nominal value unless otherwise indicated or inferred from the context.

It should be understood that the order of steps or order for performing certain actions is immaterial so long as the present disclosure remains operable. Moreover, two or more steps or actions may be conducted simultaneously.

At various places in the present specification, variables or parameters are disclosed in groups or in ranges. It is specifically intended that the description include each and every individual subcombination of the members of such groups and ranges. For example, an integer in the range of 0 to 40 is specifically intended to individually disclose 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, and 40, and an integer in the range of 1 to 20 is specifically intended to individually disclose 1, 2, 3, 4, 5, 6, 7, 8, 9, 10, 11, 12, 13, 14, 15, 16, 17, 18, 19, and 20.

The use of any and all examples, or exemplary language herein, for example, "such as" or "including," is intended merely to illustrate better the present disclosure and does not pose a limitation on the scope of the disclosure unless claimed. No language in the specification should be construed as indicating any non-claimed element as essential to the practice of the present disclosure.

As a general matter, compositions specifying a percentage are by weight unless otherwise specified. Further, if a variable is not accompanied by a definition, then the previous definition of the variable controls.

As used herein, "pharmaceutical composition" or "pharmaceutical formulation" refers to the combination of an active agent with a carrier, inert or active, making the composition especially suitable for diagnostic or therapeutic use *in vivo* or *ex vivo.*

The phrases "pharmaceutically acceptable" and "pharmacologically acceptable," as used herein, refer to compounds, molecular entities, compositions, materials, and/or dosage forms that do not produce an adverse, allergic or other untoward reaction when administered to an animal, or a human, as appropriate. For human administration, preparations should meet sterility, pyrogenicity, and general safety and purity standards as required by regulatory agencies that evaluate the safety and efficacy of pharmaceuticals and drug products, e.g., the U.S. Food and Drug Administration. "Pharmaceutically acceptable" and "pharmacologically acceptable" can mean approved or approvable by a regulatory agency of the federal or a state government or the corresponding agency in countries other than the United States, or that is listed in the U.S. Pharmacopoeia or other generally recognized pharmacopoeia for use in animals, and more particularly, in humans.

As used herein, "pharmaceutically acceptable salt" refers to any salt of an acidic or a basic group that may be present in a compound of the present disclosure (e.g., bempedoic acid), which salt is compatible with pharmaceutical administration. For example, one or both of the carboxylic acid groups of bempedoic acid can be transformed to pharmaceutically acceptable salt(s).

As is known to those of skill in the art, "salts" of compounds may be derived from inorganic or organic acids and bases. Examples of acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, naphthalene-2-sulfonic and benzenesulfonic acid. Other acids, such as oxalic, while not in themselves pharmaceutically acceptable, may be employed in the preparation of salts useful as intermediates in obtaining the compounds described herein and their pharmaceutically acceptable acid addition salts.

Examples of bases include, but are not limited to, alkali metal (e.g., sodium and potassium) hydroxides, alkaline earth metal (e.g., magnesium and calcium) hydroxides, ammonia, and compounds of formula NW₄⁺, wherein W is C₁₋₄ alkyl, and the like.

Examples of salts include, but are not limited, to acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, phenylpropionate, picrate, pivalate, propionate, succinate, tartrate, thiocyanate, tosylate, undecanoate, and the like. Other examples of salts include anions of the compounds of the present disclosure compounded with a suitable cation such as Na⁺, K⁺, Ca²⁺, NH₄⁺, and NW₄⁺ (where W can be a C₁₋₄ alkyl group), and the like.

For therapeutic use, salts of the compounds of the present disclosure are contemplated as being pharmaceutically acceptable. However, salts of acids and bases that are non-pharmaceutically acceptable may also find use, for example, in the preparation or purification of a pharmaceutically acceptable compound.

As used herein, "carrier" refers to a material, composition or vehicle, such as a liquid or solid filler, diluent, excipient, solvent or encapsulating material, involved in carrying or transporting a pharmaceutical agent such as bempedoic acid, or a pharmaceutically acceptable salt thereof, from one organ, or portion of the body, to another organ, or portion of the body.

As used herein, "pharmaceutically acceptable excipient" refers to a substance that aids the administration of an active agent to and/or absorption by a subject and can be included in the compositions of the present disclosure without causing a significant adverse toxicological effect on the patient. Non-limiting examples of pharmaceutically acceptable excipients include water, NaCl, normal saline solutions, such as a phosphate buffered saline solution, emulsions (e.g., such as an oil/water or water/oil emulsions), lactated Ringer's solution, normal sucrose, normal glucose, binders, fillers, disintegrants, lubricants, coatings, sweeteners, flavors, salt solutions (such as Ringer's solution), alcohols, oils, gelatins, carbohydrates such as lactose, amylose or starch, fatty acid esters, hydroxypropylmethylcellulose, polyvinyl pyrrolidine, and colors, and the like. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like that do not deleteriously react with the compounds of the disclosure. For examples of excipients, *see* Martin, Remington's Pharmaceutical Sciences, 15th Ed., Mack Publ. Co., Easton, PA (1975).

As used herein, "treat," "treating" or "treatment" includes an action that occurs while a subject is suffering from a specified disease, disorder or condition, which reduces the severity of the disease, disorder or condition, or retards or slows the progression of the disease, disorder or condition (e.g., lessening, reducing, modulating, ameliorating or eliminating, that results in the improvement of the condition, disease, disorder, and the like), or lessens, reduces, modulates, ameliorates or eliminates a symptom thereof. Treating can be curing, improving, or at least partially ameliorating the disorder. In certain embodiments, treating is curing the disease.

As used herein, "reducing" or "reduction" of a symptom or symptoms (and grammatical equivalents of this phrase) refers to decreasing of the severity or frequency of the symptom(s), or elimination of the symptom(s). For example, "reducing" or "reduction" of polycystic kidney disease may refer to a decrease in the rate of kidney cyst growth or a decrease in the rate of loss of kidney function.

As used herein, "reducing" or "reduction" of an elevated laboratory biomarker/parameter or vital sign associated with a disease disclosed herein may refer a decrease in the elevated laboratory biomarker/parameter or vital sign, for example, to a predetermined clinically relevant endpoint (e.g., a clinically normal level).

As used herein, "subject" and "patient" are used interchangeably and refer to an organism to be treated by the methods and compositions of the present disclosure. Such organisms are preferably a mammal (e.g., human, mouse, rat, guinea pig, dog, cat, horse, cow, pig, or non-human primate, such as a monkey, chimpanzee, baboon, and rhesus), and more preferably, a human.

As used herein, "solid dosage form" refers to a pharmaceutical dose(s) in solid form, e.g., tablets, capsules, granules, powders, sachets, reconstitutable powders, dry powder inhalers and chewables.

As used herein, "immediate release" refers to a dosage form that has not been engineered to modify or control the release of the active ingredient.

As used herein, "sustained release" refers to a dosage form designed to release a drug at a predetermined (but not necessarily constant) rate in order to maintain a desired range of drug concentration over a specific period of time, e.g., 8 hours, 12 hours, 16 hours, 20 hours, 24 hours, etc., with minimum side effects. This can be achieved through a variety of formulations, as exemplified by the bempedoic acid sustained release formulations as described in international publication WO 2019/161307A1.

As used herein, "fixed-dose combination" refers to a form in which the active ingredients (e.g., bempedoic acid and tolvaptan) are both administered to a patient simultaneously in the form of a single entity or dosage.

As used herein, "administering" refers to oral administration, administration as a suppository, topical contact, intravenous administration, parenteral administration, intraperitoneal administration, intramuscular administration, intralesional administration, intrathecal administration, intracranial administration, intranasal administration or subcutaneous administration, or the implantation of a slow-release device, e.g., a mini-osmotic pump, to a subject. Administration is by any route, including parenteral and transmucosal (e.g., buccal, sublingual, palatal, gingival, nasal, vaginal, rectal, or transdermal). Parenteral administration includes, e.g., intravenous, intramuscular, intraarterial, intradermal, subcutaneous, intraperitoneal, intraventricular, and intracranial. Other modes of delivery include, but are not limited to, the use of liposomal formulations, intravenous infusion, transdermal patches, etc. By "co-administer" it is meant that a composition described herein is administered at the same time, just prior to, or just after the administration of one or more additional therapies (e.g., bempedoic acid and tolvaptan). Tolvaptan, or a pharmaceutically acceptable salt thereof, can be administered alone or can be co-administered to the patient. Co-administration is meant to include simultaneous or sequential administration of the compound individually or in combination (more than one compound or agent). Thus, the preparations can also be combined, when desired, with other active substances (e.g., to reduce metabolic degradation).

As used herein, "disease," "disorder," "condition," or "illness," which can be used interchangeably herein unless otherwise understood from the context, refers to a state of being or health status of a patient or subject capable of being treated with a compound, pharmaceutical material, pharmaceutical composition, or method provided herein.

As used herein, "effective amount" or "therapeutically-effective amount" refers to the amount of a compound (e.g., bempedoic acid or tolvaptan), a combination of compounds (e.g., bempedoic acid and tolvaptan), a pharmaceutical composition (e.g., a pharmaceutical composition of the present disclosure), or a fixed-dose combination (e.g., a fixed-dose combination of the present disclosure) sufficient to effect beneficial or desired results. An effective amount can be administered in one or more administrations, applications or dosages and is not intended to be limited to a particular formulation or administration route.

### Abbreviations:

- ADPKD: autosomal dominant polycystic kidney disease
- PKD: polycystic kidney disease
- ACL/pACLY: ATP citrate lyase/phosphorylated ATP citrate lyase
- ACLY: ATP citrate lyase
- FATP2: fatty acid transport protein 2
- ACSVL1: very long-chain acyl-CoA synthetase
- BUN: blood urea nitrogen
- TKV: total kidney volume
- eGFR: estimated glomerular filtration rate
- KIM-1: kidney injury molecule-1
- AMPK: AMP-activated protein kinase
- Thr¹⁷²/pThr¹⁷²: phosphorylated AMPK α
- P70S6K/pP70S6K: ribosomal protein S6 kinase beta-1/phosphorylated ribosomal protein
- S6: kinase beta-1
- ERK/pERK: extracellular signal-regulated kinase/phosphorylated extracellular
- signal-: regulated kinase
- PKD1: polycystin 1
- PT: proximal tubule
- IMCD: inner medullary collecting duct
- BA: bempedoic acid
- HDL-C: high-density lipoprotein cholesterol
- CRP: C-reactive protein
- LDL-C: low-density lipoprotein-cholesterol
- VLDL: very-low-density lipoprotein
- A1c/hbA1c: glycated hemoglobin
- hsCRP: high-sensitivity C-reactive protein
- apo B: apolipoprotein B
- apo A1: apolipoprotein A1

### Bempedoic Acid

Bempedoic acid is a non-statin drug indicated as an adjunct to diet and maximally tolerated statin therapy for the treatment of adults with heterozygous familial hypercholesterolemia or established atherosclerotic cardiovascular disease who require additional lowering of LDL-C. It functions through inhibition of adenosine triphosphate-citrate lyase (ACL). It behaves as a prodrug *in vivo,* where it is converted to the active species bempedoic acid-CoA by endogenous liver acyl-Coenzyme (CoA) synthetase (ACS) activity. A specific ACS isozyme, very long-chain acyl-CoA synthetase (ACSVL1), is required to form the active species. Bempedoic acid can also activate the metabolic sensor AMPK.

Bempedoic acid is represented by the structure of Formula (I):

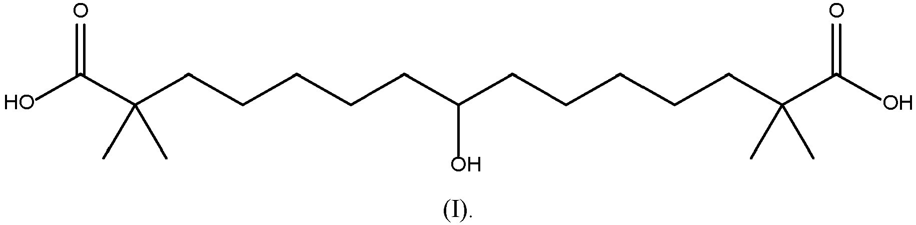

Bempedoic acid and a process for synthesizing it are disclosed in the U.S. Patent No. 7,335,799 and International Publication No. WO 2020/257571 A1, each of which is herein incorporated by reference. Bempedoic acid may also be referred to as ETC-1002, ESP-55016, or under the tradenames Nexletol^{®}, and Nilemdo^{®} (the latter of which is a fixed-dose combination of bempedoic acid and ezetimibe).

In various embodiments, bempedoic acid, or a pharmaceutically acceptable salt thereof, may be used for the treatment and/or prevention of a variety of diseases and disorders described herein. The methods of treating a disease or disorder generally comprise administering to a patient, in need thereof, a therapeutically effective amount of bempedoic acid, or a pharmaceutically acceptable salt thereof, to treat the disease or disorder.

Examples of diseases and disorders include, but are not limited to, cardiovascular disease, atrial fibrillation, blood clotting, coronary heart disease, hypercoagulable states, ischemia, myocardial infarction, myopathy, myositis, pulmonary embolism, stroke, peripheral vascular disease, dyslipidemia, dyslipoproteinemia, a disorder of glucose metabolism, Alzheimer's disease, Parkinson's disease, diabetic nephropathy, diabetic retinopathy, insulin resistance, metabolic syndrome disorders (e.g., Syndrome X), galactosemia, HIV infection, a peroxisome proliferator activated receptor-associated disorder, septicemia, a thrombotic disorder, obesity, pancreatitis, hypertension, renal disease, cancer, inflammation (e.g., liver inflammation), inflammatory muscle diseases (e.g., polymyalgia rheumatica, polymyositis, and fibrositis), impotence, gastrointestinal disease, irritable bowel syndrome, inflammatory bowel disease, inflammatory disorders (e.g., asthma, vasculitis, ulcerative colitis, Crohn's disease, Kawasaki disease, Wegener's granulomatosis, (RA), systemic lupus erythematosus (SLE), multiple sclerosis (MS), and autoimmune chronic hepatitis), arthritis (e.g., rheumatoid arthritis, juvenile rheumatoid arthritis, and osteoarthritis), osteoporosis, soft tissue rheumatism (e.g., tendonitis), bursitis, autoimmune disease (e.g., systemic lupus and erythematosus), scleroderma, ankylosing spondylitis, gout, pseudogout, non-insulin dependent diabetes mellitus, diabetes (e.g., type 2), polycystic ovarian disease, hyperlipidemias (e.g., primary hyperlipidemia, familial hypercholesterolemia (FH), Hypercholesterolemia Frederickson Type IIa, Hypercholesterolemia Frederickson Type IIb, familial combined hyperlipidemia (FCH)), lipoprotein lipase deficiencies (e.g., hypertriglyceridemia, hypoalphalipoproteinemia, and hypercholesterolemia), lipoprotein abnormalities associated with diabetes, lipoprotein abnormalities associated with obesity, and lipoprotein abnormalities associated with Alzheimer's disease. In particular embodiments, the methods include treating and/or preventing hyperlipidemia such as primary hyperlipidemia. In some embodiments, the methods include treating and/or preventing cardiovascular disease.

In certain embodiments, bempedoic acid, or a pharmaceutically acceptable salt thereof, may be used for the treatment or prevention of one or more of high levels of low density lipoprotein cholesterol (LDL-C), high levels of apolipoprotein B (apo B), high levels of lipoprotein(a) (Lp(a)), high levels of very low density lipoprotein (VLDL), high levels of non-high density lipid cholesterol (non-HDL-C), high levels of total serum cholesterol (TC), high levels of high sensitivity c-reactive protein (hsCRP), high levels of fibrinogen, high levels of insulin, high levels of glucose, and low levels of high density lipoprotein cholesterol (HDL-C). In other words, methods of the disclosure can include lowering LDL-C, lowering apo B, lowering Lp(a), lowering VLDL, lowering non-HDL-C, lowering TC, and/or lowering hsCRP. Methods of the disclosure can include inhibiting adenosine triphosphate citrate lyase (ACL), inhibiting cholesterol synthesis, and/or suppressing fatty acid biosynthesis. In some embodiments, bempedoic acid, or a pharmaceutically acceptable salt thereof, may be used for the treatment of non-insulin dependent diabetes mellitus without increasing weight gain.

In certain embodiments, bempedoic acid, or a pharmaceutically acceptable salt thereof, may be used for the treatment or prevention of a variety of diseases and conditions, which include, but are not limited to aging, Alzheimer's disease, cancer, cardiovascular disease, diabetic nephropathy, diabetic retinopathy, a disorder of glucose metabolism, dyslipidemia, dyslipoproteinemia, enhancing bile production, hypertension, impotence, inflammation, insulin resistance, lipid elimination in bile, modulating C reactive protein, obesity, oxysterol elimination in bile, pancreatitis, Parkinson's disease, a peroxisome proliferator activated receptor-associated disorder, phospholipid elimination in bile, renal disease, rhabdomyolysis, septicemia, sleep apnea, Syndrome X, and a thrombotic disorder.

In certain embodiments, the disorder is selected from the group consisting of lipodystrophy, lysosomal acid lipase deficiency, and a glycogen storage disease. In some embodiments, the patient is an adult human.

In certain embodiments, bempedoic acid, or a pharmaceutically acceptable salt thereof, may be used for the treatment or prevention of ADPKD.

In certain embodiments, the fixed-dose combinations and pharmaceutical compositions disclosed herein comprise bempedoic acid in its free-acid form.

In certain embodiments, the fixed-dose combinations and pharmaceutical compositions disclosed herein comprise a crystalline form of bempedoic acid.

In certain embodiments, the fixed-dose combinations and pharmaceutical compositions disclosed herein comprise a high purity crystalline form of bempedoic acid.

In certain embodiments, the fixed-dose combinations and pharmaceutical compositions disclosed herein comprise a pharmaceutical material comprising bempedoic acid.

In various embodiments, a pharmaceutical material generally comprises a crystalline form of the compound of formula (I), or a pharmaceutically acceptable salt thereof, wherein the pharmaceutical material comprises the compound of formula (I), or a pharmaceutically acceptable salt thereof, in an amount greater than 99.0% by weight based on the total weight of the pharmaceutical material. In some embodiments, the amount of the compound of formula (I) in the pharmaceutical material is greater than about 99.1%, greater than about 99.2%, greater than about 99.3%, greater than about 99.4%, greater than about 99.5%, greater than about 99.6%, greater than about 99.7%, greater than about 99.8%, greater than about 99.85%, greater than about 99.9%, greater than about 99.95%, or greater than about 99.98% by weight of the total weight of the pharmaceutical material. In some embodiments, the pharmaceutical material comprises the compound of formula (I) in an amount greater than 99.5% by weight based on the total weight of the pharmaceutical material. In some embodiments, the pharmaceutical material comprises the compound of formula (I) in an amount greater than 99.7% by weight based on the total weight of the pharmaceutical material. In some embodiments, the pharmaceutical material comprises the compound of formula (I) in an amount greater than 99.9% by weight based on the total weight of the pharmaceutical material.

In certain embodiments, the pharmaceutical material comprises the compound of formula (I) in an amount of from about 98% to about 102% by weight based on the total weight of the pharmaceutical material (anhydrous, solvent-free basis), as determined by a high-performance liquid chromatography (HPLC) assay.

In certain embodiments, the HPLC assay comprises one or more of:
(i) a Waters XBridge BEH C18 column (4.6 mm i.d. x 150 mm, 2.5 pm);
(ii) a column temperature of about 40 °C;
(iii) a mobile phase comprising about 0.05% phosphoric acid in water/acetonitrile (about 50:50);
(iv) isocratic elution;
(v) a flow rate of about 1.2 mL/minute;
(vi) a sample temperature of ambient temperature;
(vii) detection at 215 nm; and
(viii) the retention time of the compound of formula (I) is about 4.6 minutes. In some embodiments, the HPLC assay comprises each of the above, i.e., (i)-(viii).

In certain embodiments, the crystalline form of the compound of formula (I) may be a crystalline form of the compound of formula (I) as characterized in International Publication Nos. WO2020/257571A1 and WO2020/257573 A1. A crystalline form of the compound of formula (I) may be characterized, for example, by an X-ray powder diffraction pattern or peak(s), and/or other characteristic properties such as melting point and hygroscopicity. Crystalline forms of bempedoic acid may include, but are not limited to, cocrystals (e.g., an aspartame cocrystal and a palmitic acid cocrystal), crystalline salts (e.g., an ammonium salt, a sodium salt, a potassium salt, a calcium salt, a lysine salt, a diethylamine salt, an ethylenediamine salt, a piperazine salt, a betaine salt, a tromethamine salt, and an isonicotinamide salt).

### Tolvaptan

Tolvaptan is a vasopressin receptor 2 antagonist indicated for the treatment of patients with hypervolemic and euvolemic hyponatremia, as well as patients at risk of ADPKD. It aids in hyponatremia by promoting aquaresis, without significant sodium loss, thereby increasing sodium levels in the blood (Dubois et al; Tolvaptan; British J. of Clin. Pharmacol.; 2012; 73(1); 9-11). In ADPKD patients, tolvaptan may slow an increase in kidney volume associated with disease progression, as well as reduce pain and decline in kidney function (Sans-Atxer et al.; Tolvaptan in the treatment of autosomal dominant polycystic kidney disease; patient selection and special considerations; J. Nephrol. Renovasc. Dis.; 2018; 11: 41-51).

Tolvaptan may be used in the fixed-dose combinations, pharmaceutical compositions, and methods of treatment described herein. In various embodiments, the fixed-dose combinations and pharmaceutical compositions provided herein comprises tolvaptan, or a pharmaceutically acceptable salt thereof. Tolvaptan is represented by the structure of Formula (II):

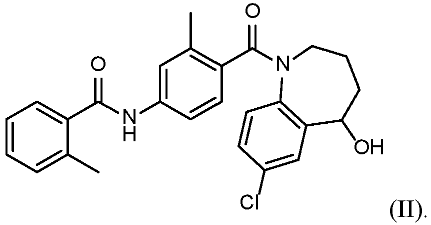

Tolvaptan and its process of manufacture are disclosed in, for example, U.S. Patent Nos. 8,501,730 and 10,905,694, which are incorporated herein by reference. Tolvaptan may be administered as an oral dosage form. Tolvaptan may also be referred to under the names of OPC-41061, Jynarque^{®} and Samsca^{®}.

In various embodiments, tolvaptan, or a pharmaceutically acceptable salt thereof, may be used for the treatment or prevention of a variety of diseases and disorders described herein. The methods of preventing or treating a disease or disorder generally comprise administering to a patient in need thereof, a therapeutically effective amount of tolvaptan, or a pharmaceutically acceptable salt thereof, to prevent or treat the disease or disorder.

Examples of diseases and disorders include, but are not limited to, hyponatremia and ADPKD.

In certain embodiments, the fixed-dose combinations and pharmaceutical compositions disclosed herein comprise tolvaptan in its free-acid/free-base form.

### Fixed-Dose Combinations

Disclosed herein are fixed-dose combinations generally comprising bempedoic acid and tolvaptan.

In some embodiments, the fixed-dose combination comprises about 30 mg to about 300 mg, about 60 mg to about 300 mg, about 90 mg to about 300 mg, about 120 mg to about 300 mg, about 150 mg to about 300 mg, about 180 mg to about 300 mg, about 210 mg to about 300 mg, about 240 mg to about 300 mg, about 270 mg to about 300 mg, about 30 mg to about 270 mg, about 60 mg to about 270 mg, about 90 mg to about 270 mg, about 120 mg to about 270 mg, about 150 mg to about 270 mg, about 180 mg to about 270 mg, about 210 mg to about 270 mg, about 240 mg to about 270 mg, about 30 mg to about 240 mg, about 60 mg to about 240 mg, about 90 mg to about 240 mg, about 120 mg to about 240 mg, about 150 mg to about 240 mg, about 180 mg to about 240 mg, about 210 mg to about 240 mg, about 30 mg to about 210 mg, about 60 mg to about 210 mg, about 90 mg to about 210 mg, about 120 mg to about 210 mg, about 150 mg to about 210 mg, about 180 mg to about 210 mg, about 30 mg to about 180 mg, about 60 mg to about 180 mg, about 90 mg to about 180 mg, about 120 mg to about 180 mg, about 150 mg to about 180 mg, about 30 mg to about 150 mg, about 60 mg to about 150 mg, about 90 mg to about 150 mg, about 120 mg to about 150 mg, about 30 mg to about 120 mg, about 60 mg to about 120 mg, about 90 mg to about 120 mg, about 30 mg to about 90 mg, about 60 mg to about 90 mg, or about 30 mg to about 60 mg bempedoic acid. In some embodiments, the fixed-dose combination comprises about 30 mg to about 240 mg. In some embodiments, the fixed-dose combination comprises about 120 mg to about 240 mg bempedoic acid.

In some embodiments, the fixed-dose combination comprises about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, or about 300 mg bempedoic acid.

In some embodiments, the fixed-dose combination comprises about 5 mg to about 120 mg, about 5 mg to about 110 mg, about 5 mg to about 100 mg, about 5 mg to about 90 mg, about 5 mg to about 80 mg, about 5 mg to about 70 mg, about 5 mg to about 60 mg, about 5 mg to about 50 mg, about 5 mg to about 40 mg, about 5 mg to about 30 mg, about 5 mg to about 20 mg, about 5 mg to about 10 mg, about 15 mg to about 90 mg, about 15 mg to about 80 mg, about 15 mg to about 70 mg, about 15 mg to about 60 mg, about 15 mg to about 50 mg, about 15 mg to about 40 mg, about 15 mg to about 30 mg, about 15 mg to about 20 mg, about 25 mg to about 90 mg, about 35 mg to about 90 mg, about 45 mg to about 90 mg, about 55 mg to about 90 mg, about 65 mg to about 90 mg, about 75 mg to about 90 mg, about 85 mg to about 90 mg, about 10 mg to about 110 mg, about 15 mg to about 105 mg, about 20 mg to about 100 mg, about 25 mg to about 95 mg, about 30 mg to about 90 mg, about 35 mg to about 85 mg, about 40 mg to about 80 mg, about 45 mg to about 75 mg, about 50 mg to about 70 mg, or about 55 mg to about 65 mg tolvaptan. In some embodiments, the fixed-dose combination comprises about 5 mg to about 120 mg tolvaptan. In some embodiments, the fixed-dose combination comprises about 5 to about 90 mg tolvaptan.

In some embodiments, the fixed-dose combination comprises about 5 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 105 mg, about 110 mg, about 115 mg, or about 120 mg tolvaptan.

In certain embodiments, the fixed-dose combination comprises about 30 mg to about 240 mg bempedoic acid and about 5 mg to about 90 mg tolvaptan. In certain embodiments, the fixed-dose combination comprises about 120 mg to about 240 mg bempedoic acid and about 5 mg to about 90 mg tolvaptan. In certain embodiments, the fixed-dose combination comprises about 30 mg to about 240 mg bempedoic acid and about 5 mg to about 60 mg tolvaptan. In certain embodiments, the fixed-dose combination comprises about 120 mg to about 240 mg bempedoic acid and about 5 mg to about 60 mg tolvaptan.

In some embodiments, the fixed-dose combination comprises about 90 mg, about 60 mg, about 45 mg, about 30 mg, about 15 mg, about 10 mg, or about 5 mg tolvaptan. In certain embodiments, the fixed-dose combination comprises about 180 mg bempedoic acid.

In particular embodiments, the fixed-dose combination comprises about 180 mg bempedoic acid and about 5 mg tolvaptan; about 180 mg bempedoic acid and about 10 mg tolvaptan; about 180 mg bempedoic acid and about 15 mg tolvaptan; about 180 mg bempedoic acid and about 30 mg tolvaptan; about 180 mg bempedoic acid and about 45 mg tolvaptan; about 180 mg bempedoic acid and about 60 mg tolvaptan; or about 180 mg bempedoic acid and about 90 mg tolvaptan.

In some embodiments, the fixed-dose combinations disclosed herein are formulated for oral delivery. In some embodiments, the fixed-dose combinations disclosed herein are formulated as an oral dosage form. Examples of oral dosage forms include, but are not limited to, a drench, a tablet, a capsule, a softgel capsule, a cachet, a pill, an emulsion, a lozenge, a solution, a suspension, a bolus, a powder, an elixir or syrup, a pastille, a mouthwash, a granule, or a paste for application to the tongue. In some embodiments, the fixed-dose combination is formulated as a tablet.

In certain embodiments, a liquid dosage form of the fixed-dose combinations described herein comprises one of the following: an inert diluent, a solubilizing agent, and an emulsifier. In certain embodiments, oral suspensions of a pharmaceutical composition of the disclosure comprise one or more suspending agents including, but not limited to, an ethoxylated isostearyl alcohol, a polyoxyethylene sorbitol and sorbitan ester, a microcrystalline cellulose, an aluminum metahydroxide, bentonite, agar-agar and tragacanth, and mixtures thereof.

In certain embodiments, the fixed-dose combinations provided herein can be for use in the treatment of a disease, disorder, or condition described herein.

### Pharmaceutical Compositions

Also disclosed herein are pharmaceutical compositions generally comprising bempedoic acid and/or tolvaptan, and one or more pharmaceutically acceptable excipients.

In certain embodiments, the pharmaceutical compositions described herein may be administered in a unit dosage form and may be prepared by any method well known in the art of pharmacy. The amounts of the compounds of bempedoic acid and tolvaptan, or pharmaceutically acceptable salts thereof, present in a single dosage form may vary depending upon the patient being treated and/or the particular mode of administration.

In certain embodiments, tolvaptan and bempedoic acid can be combined in a single pharmaceutical composition for simultaneous administration. In certain embodiments, tolvaptan and bempedoic acid can be formulated as individual pharmaceutical compositions for separate administration.

In certain embodiments, the amount of bempedoic acid and tolvaptan, or pharmaceutically acceptable salts thereof, that can be combined with a pharmaceutically acceptable carrier to produce a single dosage form will generally be an amount of the compound of bempedoic acid and tolvaptan, or pharmaceutically acceptable salts thereof, that produces a therapeutic effect.

In some embodiments, the pharmaceutical composition comprises about 30 mg to about 300 mg, about 60 mg to about 300 mg, about 90 mg to about 300 mg, about 120 mg to about 300 mg, about 150 mg to about 300 mg, about 180 mg to about 300 mg, about 210 mg to about 300 mg, about 240 mg to about 300 mg, about 270 mg to about 300 mg, about 30 mg to about 270 mg, about 60 mg to about 270 mg, about 90 mg to about 270 mg, about 120 mg to about 270 mg, about 150 mg to about 270 mg, about 180 mg to about 270 mg, about 210 mg to about 270 mg, about 240 mg to about 270 mg, about 30 mg to about 240 mg, about 60 mg to about 240 mg, about 90 mg to about 240 mg, about 120 mg to about 240 mg, about 150 mg to about 240 mg, about 180 mg to about 240 mg, about 210 mg to about 240 mg, about 30 mg to about 210 mg, about 60 mg to about 210 mg, about 90 mg to about 210 mg, about 120 mg to about 210 mg, about 150 mg to about 210 mg, about 180 mg to about 210 mg, about 30 mg to about 180 mg, about 60 mg to about 180 mg, about 90 mg to about 180 mg, about 120 mg to about 180 mg, about 150 mg to about 180 mg, about 30 mg to about 150 mg, about 60 mg to about 150 mg, about 90 mg to about 150 mg, about 120 mg to about 150 mg, about 30 mg to about 120 mg, about 60 mg to about 120 mg, about 90 mg to about 120 mg, about 30 mg to about 90 mg, about 60 mg to about 90 mg, or about 30 mg to about 60 mg bempedoic acid. In some embodiments, the pharmaceutical composition comprises about 30 mg to about 240 mg bempedoic acid. In some embodiments, the pharmaceutical composition comprises about 120 mg to about 240 mg bempedoic acid.

In some embodiments, the pharmaceutical composition comprises about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, or about 300 mg bempedoic acid.

In some embodiments, the pharmaceutical composition comprises about 5 mg to about 120 mg, about 5 mg to about 110 mg, about 5 mg to about 100 mg, about 5 mg to about 90 mg, about 5 mg to about 80 mg, about 5 mg to about 70 mg, about 5 mg to about 60 mg, about 5 mg to about 50 mg, about 5 mg to about 40 mg, about 5 mg to about 30 mg, about 5 mg to about 20 mg, about 5 mg to about 10 mg, about 15 mg to about 90 mg, about 15 mg to about 80 mg, about 15 mg to about 70 mg, about 15 mg to about 60 mg, about 15 mg to about 50 mg, about 15 mg to about 40 mg, about 15 mg to about 30 mg, about 15 mg to about 20 mg, about 25 mg to about 90 mg, about 35 mg to about 90 mg, about 45 mg to about 90 mg, about 55 mg to about 90 mg, about 65 mg to about 90 mg, about 75 mg to about 90 mg, about 85 mg to about 90 mg, about 10 mg to about 110 mg, about 15 mg to about 105 mg, about 20 mg to about 100 mg, about 25 mg to about 95 mg, about 30 mg to about 90 mg, about 35 mg to about 85 mg, about 40 mg to about 80 mg, about 45 mg to about 75 mg, about 50 mg to about 70 mg, or about 55 mg to about 65 mg tolvaptan. In some embodiments, the pharmaceutical composition comprises about 5 mg to about 120 mg tolvaptan. In some embodiments, the pharmaceutical composition comprises about 5 to about 90 mg tolvaptan.

In some embodiments, the pharmaceutical composition comprises about 5 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 105 mg, about 110 mg, about 115 mg, or about 120 mg tolvaptan.

In certain embodiments, the pharmaceutical composition comprises about 30 mg to about 240 mg bempedoic acid, about 5 mg to about 90 mg tolvaptan, and one or more pharmaceutically acceptable excipients. In certain embodiments, the pharmaceutical composition comprises about 120 mg to about 240 mg bempedoic acid, about 5 mg to about 90 mg tolvaptan, and one or more pharmaceutically acceptable excipients. In certain embodiments, the pharmaceutical composition comprises about 30 mg to about 240 mg bempedoic acid, about 5 mg to about 60 mg tolvaptan, and one or more pharmaceutically acceptable excipients. In certain embodiments, the pharmaceutical composition comprises about 120 mg to about 240 mg bempedoic acid, about 5 mg to about 60 mg tolvaptan, and one or more pharmaceutically acceptable excipients.

In some embodiments, the pharmaceutical composition comprises about 90 mg, about 60 mg, about 45 mg, about 30 mg, about 15 mg, about 10 mg, or about 5 mg tolvaptan. In certain embodiments, the pharmaceutical composition comprises about 180 mg bempedoic acid.

In particular embodiments, the pharmaceutical composition comprises about 180 mg bempedoic acid, about 5 mg tolvaptan, and one or more pharmaceutically acceptable excipients; about 180 mg bempedoic acid, about 10 mg tolvaptan, and one or more pharmaceutically acceptable excipients; about 180 mg bempedoic acid, about 15 mg tolvaptan, and one or more pharmaceutically acceptable excipients; about 180 mg bempedoic acid, about 30 mg tolvaptan, and one or more pharmaceutically acceptable excipients; about 180 mg bempedoic acid, about 45 mg tolvaptan, and one or more pharmaceutically acceptable excipients; about 180 mg bempedoic acid, about 60 mg tolvaptan, and one or more pharmaceutically acceptable excipients; or about 180 mg bempedoic acid, about 90 mg tolvaptan, and one or more pharmaceutically acceptable excipients.

In certain embodiments, a pharmaceutical composition generally comprises a fixed-dose combination described herein and one or more pharmaceutically acceptable excipients.

In certain embodiments, the solid dosage forms described herein to be used for oral administration are prepared by mixing a pharmaceutical material with one or more pharmaceutically acceptable excipients. Pharmaceutical excipients can be selected from the group consisting of a filler or extender, a sweetening agent, a binder, a humectant, a disintegrating agent, a preservative, a perfuming agent, a flavoring agent, an antioxidant, a solution retarding agent, an absorption accelerator, a wetting agent, an absorbent, a lubricant, a coloring agent, and a controlled release agent. In some embodiments, when the solid dosage form is a capsule, a tablet or a pill, the pharmaceutical compositions described herein may also comprise a buffering agent. In some embodiments, when the solid dosage form is a gelatin capsule, the pharmaceutical composition may further comprise one or more excipients selected from lactose, a milk sugar, a high molecular weight polyethylene glycol and combinations thereof.

In certain embodiments, a pharmaceutical composition of the disclosure may comprise one or more excipients selected from the group consisting of a cyclodextrin, a cellulose, a liposome, a micelle forming agent, and a polymeric carrier. In some embodiments, the pharmaceutical compositions of the present disclosure comprise an antibacterial agent, an antifungal agent, or combinations thereof. Examples of antibacterial and antifungal agents include, but are not limited to, paraben, chlorobutanol, phenol and sorbic acid. In some embodiments, the pharmaceutical compositions of the present disclosure comprise an isotonic agent.

In some embodiments, the pharmaceutical compositions disclosed herein are formulated for oral delivery. In some embodiments, the pharmaceutical compositions disclosed herein are formulated as an oral dosage form. Examples of oral dosage forms include, but are not limited to a drench, a tablet, a capsule, a softgel capsule, a cachet, a pill, an emulsion, a lozenge, a solution, a suspension, a bolus, a powder, an elixir or syrup, a pastille, a mouthwash, a granule, or a paste for application to the tongue. In some embodiments, the pharmaceutical composition is formulated as a tablet.

In certain embodiments, a liquid dosage form of the pharmaceutical compositions described herein comprises one of the following: an inert diluent, a solubilizing agent, and an emulsifier. In certain embodiments, oral suspensions of a pharmaceutical composition of the disclosure comprise one or more suspending agents including an ethoxylated isostearyl alcohol, a polyoxyethylene sorbitol and sorbitan ester, a microcrystalline cellulose, an aluminum metahydroxide, bentonite, agar-agar and tragacanth, and mixtures thereof.

In some embodiments, the pharmaceutical compositions disclosed herein provide immediate release of bempedoic acid. In some embodiments, the pharmaceutical compositions disclosed herein provide sustained release of bempedoic acid.

In certain embodiments, a pharmaceutical composition described herein can be for use in the treatment of a disease, disorder, or condition described herein.

### Methods of Use and Treatments

Provided herein are methods of treating ADPKD in a subject receiving tolvaptan therapy, the methods comprising administering to the subject an effective amount of bempedoic acid. Also provided herein are methods of slowing the progression of ADPKD in a subject receiving tolvaptan therapy, the methods comprising administering to the subject an effective amount of bempedoic acid. Also provided herein are methods of preventing kidney failure, reducing total kidney volume, and/or slowing the progression of kidney dysfunction in a subject with ADPKD receiving tolvaptan therapy, the methods comprising administering to the subject an effective amount of bempedoic acid.

In some embodiments, the effective amount of bempedoic acid is about 30 mg to about 240 mg. In some embodiments, the effective amount of bempedoic acid is about 120 mg to about 240 mg. In certain embodiments, the effective amount of bempedoic acid is about 180 mg.

In some embodiments, the subject receiving the effective amount of bempedoic acid and tolvaptan therapy has a lower total kidney weight to body weight ratio than a subject not receiving the effective amount of bempedoic acid and tolvaptan therapy, or receiving only the effective amount of bempedoic acid, or receiving only tolvaptan therapy. In some embodiments, the subject receiving the effective amount of bempedoic acid and tolvaptan therapy has a lower blood urea nitrogen level than a subject not receiving the effective amount of bempedoic acid and tolvaptan therapy, or receiving only the effective amount of bempedoic acid, or receiving only tolvaptan therapy.

In some embodiments, the subject receiving the effective amount of bempedoic acid and tolvaptan therapy has a total kidney weight to body weight ratio about 10 to about 140 percent, about 20 to about 140 percent, about 30 to about 140 percent, about 40 to about 140 percent, about 50 to about 140 percent, about 60 to about 140 percent, about 70 to about 140 percent, about 80 to about 140 percent, about 90 to about 140 percent, about 100 to about 140 percent, about 110 to about 140 percent, about 120 to about 140 percent, about 130 to about 140 percent, about 10 to about 80 percent, about 10 to about 70 percent, about 10 to about 60 percent, about 10 to about 50 percent, about 10 to about 40 percent, about 10 to about 30 percent, about 10 to about 20 percent, about 30 to about 70 percent, about 40 to about 70 percent, about 50 to about 70 percent, about 60 to about 70 percent, about 40 to about 60 percent, about 40 to about 50 percent, about 10 to about 20 percent, about 20 to about 30 percent, about 30 to about 40 percent, about 40 to about 50 percent, about 50 percent to about 60 percent, about 60 percent to about 70 percent, about 70 percent to about 80 percent, about 80 percent to about 90 percent, about 90 to about 100 percent, about 100 to about 110 percent, about 110 to about 120 percent, about 120 to about 130 percent, or about 130 percent to about 140 percent lower than the total kidney weight to body weight ratio of a subject not receiving the effective amount of bempedoic acid and tolvaptan therapy, or receiving only the effective amount of bempedoic acid, or receiving only tolvaptan therapy.

In some embodiments, the subject receiving the effective amount of bempedoic acid and tolvaptan therapy has a blood urea nitrogen (BUN) level about 2 to about 15 mg/dL, about 3 to about 15 mg/dL, about 4 to about 15 mg/dL, about 5 to about 15 mg/dL, about 6 to about 15 mg/dL, about 7 to about 15 mg/dL, about 8 to about 15 mg/dL, about 9 to about 15 mg/dL, about 10 to about 15 mg/dL, about 11 to about 15 mg/dL, about 12 to about 15 mg/dL, about 13 to about 15 mg/dL, about 14 to about 15 mg/dL, about 2 to about 14 mg/dL, about 2 to about 13 mg/dL, about 2 to about 12 mg/dL, about 2 to about 11 mg/dL, about 2 to about 10 mg/dL, about 2 to about 9 mg/dL, about 2 to about 8 mg/dL, about 2 to about 7 mg/dL, about 2 to about 6 mg/dL, about 2 to about 5 mg/dL, about 2 to about 4 mg/dL, about 2 to about 3 mg/dL, about 3 to about 9 mg/dL, about 4 to about 9 mg/dL, about 5 to about 9 mg/dL, about 6 to about 9 mg/dL, about 7 to about 9 mg/dL, about 8 to about 9 mg/dL, about 3 to about 8 mg/dL, about 3 to about 7 mg/dL, about 3 to about 6 mg/dL, about 3 to about 5 mg/dL, or about 3 to about 4 mg/dL lower than the BUN of a subject not receiving the effective amount of bempedoic acid and tolvaptan therapy, or receiving only the effective amount of bempedoic acid, or receiving only tolvaptan therapy.

In some embodiments, the subject receiving the effective amount of bempedoic acid and tolvaptan therapy has a decrease in estimated glomerular filtration rate (eGFR) of about 0.5 to about 5 mL/min/1.73m²/year, about 1 to about 5 mL/min/1.73m²/year, about 1.5 to about 5 mL/min/1.73m²/year, about 2 to about 5 mL/min/1.73m²/year, about 2.5 to about 5 mL/min/1.73m²/year, about 3 to about 5 mL/min/1.73m²/year, about 3.5 to about 5 mL/min/1.73m²/year, about 4 to about 5 mL/min/1.73m²/year, about 4.5 to about 5 mL/min/1.73m²/year, about 0.5 to about 4.5 mL/min/1.73m²/year, about 0.5 to about 4 mL/min/1.73m²/year, about 0.5 to about 3.5 mL/min/1.73m²/year, about 0.5 to about 3 mL/min/1.73m²/year, about 0.5 to about 2.5 mL/min/1.73m²/year, about 0.5 to about 2 mL/min/1.73m²/year, about 0.5 to about 1.5 mL/min/1.73m²/year, about 0.5 to about 1 mL/min/1.73m²/year, about 1.7 to about 3.4 mL/min/1.73m²/year, about 1.9 to about 3.4 mL/min/1.73m²/year, about 2.1 to about 3.4 mL/min/1.73m²/year, about 2.3 to about 3.4 mL/min/1.73m²/year, about 2.5 to about 3.4 mL/min/1.73m²/year, about 2.7 to about 3.4 mL/min/1.73m²/year, about 2.9 to about 3.4 mL/min/1.73m²/year, about 3.1 to about 3.4 mL/min/1.73m²/year, about 3.3 to about 3.4 mL/min/1.73m²/year, about 1.7 to about 1.9 mL/min/1.73m²/year, about 1.9 to about 2.1 mL/min/1.73m²/year, about 2.1 to about 2.3 mL/min/1.73m²/year, about 2.3 to about 2.5 mL/min/1.73m²/year, about 2.5 to about 2.7 mL/min/1.73m²/year, about 2.7 to about 2.9 mL/min/1.73m²/year, about 2.9 to about 3.1 mL/min/1.73m²/year, about 3.1 to about 3.3 mL/min/1.73m²/year, or about 3.3 to about 3.4 mL/min/1.73m²/year lower than the decrease in eGFR per year of a subject not receiving the effective amount of bempedoic acid and tolvaptan therapy, or receiving only the effective amount of bempedoic acid, or receiving only tolvaptan therapy.

In some embodiments, the subject receiving the effective amount of bempedoic acid and tolvaptan therapy has an increase in total kidney volume of about 0 to about 7 percent/year, about 0.5 to about 7 percent/year, about 1 to about 7 percent/year, about 1.5 to about 7 percent/year, about 2 to about 7 percent/year, about 2.5 to about 7 percent/year, about 3 to about 7 percent/year, about 3.5 to about 7 percent/year, about 4 to about 7 percent/year, about 4.5 to about 7 percent/year, about 5 to about 7 percent/year, about 5.5 to about 7 percent/year, about 6 to about 7 percent/year, about 6.5 to about 7 percent/year, about 0 to about 6.5 percent/year, about 0 to about 6 percent/year, about 0 to about 5.5 percent/year, about 0 to about 5 percent/year, about 0 to about 4.5 percent/year, about 0 to about 4 percent/year, about 0 to about 3.5 percent/year, about 0 to about 3 percent/year, about 0 to about 2.5 percent/year, about 0 to about 2 percent/year, about 0 to about 1.5 percent/year, about 0 to about 1 percent/year, about 0 to about 0.5 percent/year, about 1 to about 5 percent/year, about 1.5 to about 5 percent/year, about 2 to about 5 percent/year, about 2.5 to about 5 percent/year, about 3 to about 5 percent/year, about 3.5 to about 5 percent/year, about 4 to about 5 percent/year, about 4.5 to about 5 percent/year, about 1 to about 1.5 percent/year, about 1.5 to about 2 percent/year, about 2 to about 2.5 percent/year, about 2.5 to about 3 percent/year, about 3 to about 3.5 percent/year, about 3.5 to about 4 percent/year, about 4 to about 4.5 percent/year, or about 4.5 to about 5 percent/year lower than the percent/year increase in total kidney volume of a subject not receiving the effective amount of bempedoic acid and tolvaptan therapy, or receiving only the effective amount of bempedoic acid, or receiving only tolvaptan therapy.

In some embodiments, the subject receiving the effective amount of bempedoic acid and tolvaptan therapy has a composite incidence of clinical endpoints selected from worsening kidney function, kidney pain, hypertension, and albuminuria of about 5 to about 50 percent, about 10 to about 50 percent, about 15 to about 50 percent, about 20 to about 50 percent, about 25 to about 50 percent, about 30 to about 50 percent, about 35 to about 50 percent, about 40 to about 50 percent, about 45 to about 50 percent, about 5 to about 45 percent, about 5 to about 40 percent, about 5 to about 35 percent, about 5 to about 30 percent, about 5 to about 25 percent, about 5 to about 20 percent, about 5 to about 15 percent, about 5 to about 10 percent, about 15 to about 40 percent, about 20 to about 40 percent, about 25 to about 40 percent, about 30 to about 40 percent, about 35 to about 40 percent, about 15 to about 20 percent, about 20 to about 25 percent, about 25 to about 30 percent, about 30 to about 35 percent, or about 35 to about 40 percent lower than the composite incidence of clinical endpoints of a subject not receiving the effective amount of bempedoic acid and tolvaptan therapy, or receiving only the effective amount of bempedoic acid, or receiving only tolvaptan therapy.

In some embodiments, the subject receiving the effective amount of bempedoic acid and tolvaptan therapy has higher liver AMPK activity than a subject not receiving the effective amount of bempedoic acid, or receiving only tolvaptan therapy. In some embodiments, the subject receiving the effective amount of bempedoic acid and tolvaptan therapy has a higher degree of liver acetyl-coenzyme A carboxylase (ACC) phosphorylation than a subject not receiving the effective amount of bempedoic acid, or receiving only tolvaptan therapy. In some embodiments, the subject receiving the effective amount of bempedoic acid and tolvaptan therapy has a higher degree of peroxisome proliferator-activated receptor gamma coactivator 1-α (PGC1α) activity than a subject receiving only tolvaptan therapy.

In some embodiments, the effective amount of bempedoic acid is delivered orally. Examples of oral dosage forms include, but are not limited to a drench, a tablet, a capsule, a softgel capsule, a cachet, a pill, an emulsion, a lozenge, a solution, a suspension, a bolus, a powder, an elixir or syrup, a pastille, a mouthwash, a granule, or a paste for application to the tongue. In some embodiments, the pharmaceutical composition is formulated as a tablet.

Also provided herein are methods of treating ADPKD in a subject in need thereof, the methods comprising administering to the subject a fixed-dose combination of bempedoic acid and tolvaptan, wherein the fixed-dose combination comprises about 30 mg to about 240 mg bempedoic acid and about 5 mg to about 90 mg tolvaptan. Also provided herein are methods of treating ADPKD in a subject in need thereof, the methods comprising administering to the subject a fixed-dose combination of bempedoic acid and tolvaptan, wherein the fixed-dose combination comprises about 120 mg to about 240 mg bempedoic acid and about 5 mg to about 90 mg tolvaptan. Also provided herein are methods of slowing the progression of ADPKD in a subject in need thereof, the methods comprising administering to the subject a fixed-dose combination of bempedoic acid and tolvaptan, wherein the fixed-dose combination comprises about 30 mg to about 240 mg bempedoic acid and about 5 mg to about 90 mg tolvaptan. Also provided herein are methods of slowing the progression of ADPKD in a subject in need thereof, the methods comprising administering to the subject a fixed-dose combination of bempedoic acid and tolvaptan, wherein the fixed-dose combination comprises about 120 mg to about 240 mg bempedoic acid and about 5 mg to about 90 mg tolvaptan. Also provided herein are methods of preventing kidney failure, reducing total kidney volume, and/or slowing the progression of kidney dysfunction in a subject with ADPKD, the methods comprising administering to the subject a fixed-dose combination of bempedoic acid and tolvaptan, wherein the fixed-dose combination comprises about 30 mg to about 240 mg bempedoic acid and about 5 mg to about 90 mg tolvaptan. Also provided herein are methods of preventing kidney failure, reducing total kidney volume, and/or slowing the progression of kidney dysfunction in a subject with ADPKD, the methods comprising administering to the subject a fixed-dose combination of bempedoic acid and tolvaptan, wherein the fixed-dose combination comprises about 120 mg to about 240 mg bempedoic acid and about 5 mg to about 90 mg tolvaptan.

In some embodiments, the subject receiving the fixed-dose combination has a lower total kidney weight to body weight ratio than a subject not receiving the fixed-dose combination, or receiving only about 30 mg to about 240 mg bempedoic acid, or receiving only about 5 mg to about 90 mg tolvaptan. In some embodiments, the subject receiving the fixed-dose combination has a lower total kidney weight to body weight ratio than a subject not receiving the fixed-dose combination, or receiving only about 120 mg to about 240 mg bempedoic acid, or receiving only about 5 mg to about 90 mg tolvaptan. In some embodiments, the subject receiving the fixed-dose combination has a lower blood urea nitrogen level than a subject not receiving the fixed-dose combination, or receiving only about 30 mg to about 240 mg bempedoic acid, or receiving only about 5 mg to about 90 mg tolvaptan. In some embodiments, the subject receiving the fixed-dose combination has a lower blood urea nitrogen level than a subject not receiving the fixed-dose combination, or receiving only about 120 mg to about 240 mg bempedoic acid, or receiving only about 5 mg to about 90 mg tolvaptan.

In some embodiments, the subject receiving the fixed-dose combination has a total kidney weight to body weight ratio about 10 to about 140 percent, about 20 to about 140 percent, about 30 to about 140 percent, about 40 to about 140 percent, about 50 to about 140 percent, about 60 to about 140 percent, about 70 to about 140 percent, about 80 to about 140 percent, about 90 to about 140 percent, about 100 to about 140 percent, about 110 to about 140 percent, about 120 to about 140 percent, about 130 to about 140 percent, about 10 to about 80 percent, about 10 to about 70 percent, about 10 to about 60 percent, about 10 to about 50 percent, about 10 to about 40 percent, about 10 to about 30 percent, about 10 to about 20 percent, about 30 to about 70 percent, about 40 to about 70 percent, about 50 to about 70 percent, about 60 to about 70 percent, about 40 to about 60 percent, about 40 to about 50 percent, about 10 to about 20 percent, about 20 to about 30 percent, about 30 to about 40 percent, about 40 to about 50 percent, about 50 percent to about 60 percent, about 60 percent to about 70 percent, about 70 percent to about 80 percent, about 80 percent to about 90 percent, about 90 to about 100 percent, about 100 to about 110 percent, about 110 to about 120 percent, about 120 to about 130 percent, or about 130 percent to about 140 percent lower than the total kidney weight to body weight ratio of a subject not receiving the fixed-dose combination, or receiving only about 30 mg to about 240 mg bempedoic acid, or receiving only about 5 mg to about 90 mg tolvaptan.

In some embodiments, the subject receiving the fixed-dose combination has a total kidney weight to body weight ratio about 10 to about 140 percent, about 20 to about 140 percent, about 30 to about 140 percent, about 40 to about 140 percent, about 50 to about 140 percent, about 60 to about 140 percent, about 70 to about 140 percent, about 80 to about 140 percent, about 90 to about 140 percent, about 100 to about 140 percent, about 110 to about 140 percent, about 120 to about 140 percent, about 130 to about 140 percent, about 10 to about 80 percent, about 10 to about 70 percent, about 10 to about 60 percent, about 10 to about 50 percent, about 10 to about 40 percent, about 10 to about 30 percent, about 10 to about 20 percent, about 30 to about 70 percent, about 40 to about 70 percent, about 50 to about 70 percent, about 60 to about 70 percent, about 40 to about 60 percent, about 40 to about 50 percent, about 10 to about 20 percent, about 20 to about 30 percent, about 30 to about 40 percent, about 40 to about 50 percent, about 50 percent to about 60 percent, about 60 percent to about 70 percent, about 70 percent to about 80 percent, about 80 percent to about 90 percent, about 90 to about 100 percent, about 100 to about 110 percent, about 110 to about 120 percent, about 120 to about 130 percent, or about 130 percent to about 140 percent lower than the total kidney weight to body weight ratio of a subject not receiving the fixed-dose combination, or receiving only about 120 mg to about 240 mg bempedoic acid, or receiving only about 5 mg to about 90 mg tolvaptan.

In some embodiments, the subject receiving the fixed-dose combination has a BUN level about 2 to about 15 mg/dL, about 3 to about 15 mg/dL, about 4 to about 15 mg/dL, about 5 to about 15 mg/dL, about 6 to about 15 mg/dL, about 7 to about 15 mg/dL, about 8 to about 15 mg/dL, about 9 to about 15 mg/dL, about 10 to about 15 mg/dL, about 11 to about 15 mg/dL, about 12 to about 15 mg/dL, about 13 to about 15 mg/dL, about 14 to about 15 mg/dL, about 2 to about 14 mg/dL, about 2 to about 13 mg/dL, about 2 to about 12 mg/dL, about 2 to about 11 mg/dL, about 2 to about 10 mg/dL, about 2 to about 9 mg/dL, about 2 to about 8 mg/dL, about 2 to about 7 mg/dL, about 2 to about 6 mg/dL, about 2 to about 5 mg/dL, about 2 to about 4 mg/dL, about 2 to about 3 mg/dL, about 3 to about 9 mg/dL, about 4 to about 9 mg/dL, about 5 to about 9 mg/dL, about 6 to about 9 mg/dL, about 7 to about 9 mg/dL, about 8 to about 9 mg/dL, about 3 to about 8 mg/dL, about 3 to about 7 mg/dL, about 3 to about 6 mg/dL, about 3 to about 5 mg/dL, or about 3 to about 4 mg/dL lower than the BUN of a subject not receiving the fixed-dose combination, or receiving only about 30 mg to about 240 mg bempedoic acid, or receiving only about 5 mg to about 90 mg tolvaptan.

In some embodiments, the subject receiving the fixed-dose combination has a BUN level about 2 to about 15 mg/dL, about 3 to about 15 mg/dL, about 4 to about 15 mg/dL, about 5 to about 15 mg/dL, about 6 to about 15 mg/dL, about 7 to about 15 mg/dL, about 8 to about 15 mg/dL, about 9 to about 15 mg/dL, about 10 to about 15 mg/dL, about 11 to about 15 mg/dL, about 12 to about 15 mg/dL, about 13 to about 15 mg/dL, about 14 to about 15 mg/dL, about 2 to about 14 mg/dL, about 2 to about 13 mg/dL, about 2 to about 12 mg/dL, about 2 to about 11 mg/dL, about 2 to about 10 mg/dL, about 2 to about 9 mg/dL, about 2 to about 8 mg/dL, about 2 to about 7 mg/dL, about 2 to about 6 mg/dL, about 2 to about 5 mg/dL, about 2 to about 4 mg/dL, about 2 to about 3 mg/dL, about 3 to about 9 mg/dL, about 4 to about 9 mg/dL, about 5 to about 9 mg/dL, about 6 to about 9 mg/dL, about 7 to about 9 mg/dL, about 8 to about 9 mg/dL, about 3 to about 8 mg/dL, about 3 to about 7 mg/dL, about 3 to about 6 mg/dL, about 3 to about 5 mg/dL, or about 3 to about 4 mg/dL lower than the BUN of a subject not receiving the fixed-dose combination, or receiving only about 120 mg to about 240 mg bempedoic acid, or receiving only about 5 mg to about 90 mg tolvaptan.

In some embodiments, the subject receiving the fixed-dose combination has a decrease in eGFR of about 0.5 to about 5 mL/min/1.73m²/year, about 1 to about 5 mL/min/1.73m²/year, about 1.5 to about 5 mL/min/1.73m²/year, about 2 to about 5 mL/min/1.73m²/year, about 2.5 to about 5 mL/min/1.73m²/year, about 3 to about 5 mL/min/1.73m²/year, about 3.5 to about 5 mL/min/1.73m²/year, about 4 to about 5 mL/min/1.73m²/year, about 4.5 to about 5 mL/min/1.73m²/year, about 0.5 to about 4.5 mL/min/1.73m²/year, about 0.5 to about 4 mL/min/1.73m²/year, about 0.5 to about 3.5 mL/min/1.73m²/year, about 0.5 to about 3 mL/min/1.73m²/year, about 0.5 to about 2.5 mL/min/1.73m²/year, about 0.5 to about 2 mL/min/1.73m²/year, about 0.5 to about 1.5 mL/min/1.73m²/year, about 0.5 to about 1 mL/min/1.73m²/year, about 1.7 to about 3.4 mL/min/1.73m²/year, about 1.9 to about 3.4 mL/min/1.73m²/year, about 2.1 to about 3.4 mL/min/1.73m²/year, about 2.3 to about 3.4 mL/min/1.73m²/year, about 2.5 to about 3.4 mL/min/1.73m²/year, about 2.7 to about 3.4 mL/min/1.73m²/year, about 2.9 to about 3.4 mL/min/1.73m²/year, about 3.1 to about 3.4 mL/min/1.73m²/year, about 3.3 to about 3.4 mL/min/1.73m²/year, about 1.7 to about 1.9 mL/min/1.73m²/year, about 1.9 to about 2.1 mL/min/1.73m²/year, about 2.1 to about 2.3 mL/min/1.73m²/year, about 2.3 to about 2.5 mL/min/1.73m²/year, about 2.5 to about 2.7 mL/min/1.73m²/year, about 2.7 to about 2.9 mL/min/1.73m²/year, about 2.9 to about 3.1 mL/min/1.73m²/year, about 3.1 to about 3.3 mL/min/1.73m²/year, or about 3.3 to about 3.4 mL/min/1.73m²/year lower than the decrease in eGFR per year of a subject not receiving the fixed-dose combination, or receiving only about 30 mg to about 240 mg bempedoic acid, or receiving only about 5 mg to about 90 mg tolvaptan.

In some embodiments, the subject receiving the fixed-dose combination has a decrease in eGFR of about 0.5 to about 5 mL/min/1.73m²/year, about 1 to about 5 mL/min/1.73m²/year, about 1.5 to about 5 mL/min/1.73m²/year, about 2 to about 5 mL/min/1.73m²/year, about 2.5 to about 5 mL/min/1.73m²/year, about 3 to about 5 mL/min/1.73m²/year, about 3.5 to about 5 mL/min/1.73m²/year, about 4 to about 5 mL/min/1.73m²/year, about 4.5 to about 5 mL/min/1.73m²/year, about 0.5 to about 4.5 mL/min/1.73m²/year, about 0.5 to about 4 mL/min/1.73m²/year, about 0.5 to about 3.5 mL/min/1.73m²/year, about 0.5 to about 3 mL/min/1.73m²/year, about 0.5 to about 2.5 mL/min/1.73m²/year, about 0.5 to about 2 mL/min/1.73m²/year, about 0.5 to about 1.5 mL/min/1.73m²/year, about 0.5 to about 1 mL/min/1.73m²/year, about 1.7 to about 3.4 mL/min/1.73m²/year, about 1.9 to about 3.4 mL/min/1.73m²/year, about 2.1 to about 3.4 mL/min/1.73m²/year, about 2.3 to about 3.4 mL/min/1.73m²/year, about 2.5 to about 3.4 mL/min/1.73m²/year, about 2.7 to about 3.4 mL/min/1.73m²/year, about 2.9 to about 3.4 mL/min/1.73m²/year, about 3.1 to about 3.4 mL/min/1.73m²/year, about 3.3 to about 3.4 mL/min/1.73m²/year, about 1.7 to about 1.9 mL/min/1.73m²/year, about 1.9 to about 2.1 mL/min/1.73m²/year, about 2.1 to about 2.3 mL/min/1.73m²/year, about 2.3 to about 2.5 mL/min/1.73m²/year, about 2.5 to about 2.7 mL/min/1.73m²/year, about 2.7 to about 2.9 mL/min/1.73m²/year, about 2.9 to about 3.1 mL/min/1.73m²/year, about 3.1 to about 3.3 mL/min/1.73m²/year, or about 3.3 to about 3.4 mL/min/1.73m²/year lower than the decrease in eGFR per year of a subject not receiving the fixed-dose combination, or receiving only about 120 mg to about 240 mg bempedoic acid, or receiving only about 5 mg to about 90 mg tolvaptan.

In some embodiments, the subject receiving the fixed-dose combination has an increase in total kidney volume of about 0 to about 7 percent/year, about 0.5 to about 7 percent/year, about 1 to about 7 percent/year, about 1.5 to about 7 percent/year, about 2 to about 7 percent/year, about 2.5 to about 7 percent/year, about 3 to about 7 percent/year, about 3.5 to about 7 percent/year, about 4 to about 7 percent/year, about 4.5 to about 7 percent/year, about 5 to about 7 percent/year, about 5.5 to about 7 percent/year, about 6 to about 7 percent/year, about 6.5 to about 7 percent/year, about 0 to about 6.5 percent/year, about 0 to about 6 percent/year, about 0 to about 5.5 percent/year, about 0 to about 5 percent/year, about 0 to about 4.5 percent/year, about 0 to about 4 percent/year, about 0 to about 3.5 percent/year, about 0 to about 3 percent/year, about 0 to about 2.5 percent/year, about 0 to about 2 percent/year, about 0 to about 1.5 percent/year, about 0 to about 1 percent/year, about 0 to about 0.5 percent/year, about 1 to about 5 percent/year, about 1.5 to about 5 percent/year, about 2 to about 5 percent/year, about 2.5 to about 5 percent/year, about 3 to about 5 percent/year, about 3.5 to about 5 percent/year, about 4 to about 5 percent/year, about 4.5 to about 5 percent/year, about 1 to about 1.5 percent/year, about 1.5 to about 2 percent/year, about 2 to about 2.5 percent/year, about 2.5 to about 3 percent/year, about 3 to about 3.5 percent/year, about 3.5 to about 4 percent/year, about 4 to about 4.5 percent/year, or about 4.5 to about 5 percent/year lower than the percent/year increase in total kidney volume of a subject not receiving the fixed-dose combination, or receiving only about 30 mg to about 240 mg bempedoic acid, or receiving only about 5 mg to about 90 mg tolvaptan.

In some embodiments, the subject receiving the fixed-dose combination has an increase in total kidney volume of about 0 to about 7 percent/year, about 0.5 to about 7 percent/year, about 1 to about 7 percent/year, about 1.5 to about 7 percent/year, about 2 to about 7 percent/year, about 2.5 to about 7 percent/year, about 3 to about 7 percent/year, about 3.5 to about 7 percent/year, about 4 to about 7 percent/year, about 4.5 to about 7 percent/year, about 5 to about 7 percent/year, about 5.5 to about 7 percent/year, about 6 to about 7 percent/year, about 6.5 to about 7 percent/year, about 0 to about 6.5 percent/year, about 0 to about 6 percent/year, about 0 to about 5.5 percent/year, about 0 to about 5 percent/year, about 0 to about 4.5 percent/year, about 0 to about 4 percent/year, about 0 to about 3.5 percent/year, about 0 to about 3 percent/year, about 0 to about 2.5 percent/year, about 0 to about 2 percent/year, about 0 to about 1.5 percent/year, about 0 to about 1 percent/year, about 0 to about 0.5 percent/year, about 1 to about 5 percent/year, about 1.5 to about 5 percent/year, about 2 to about 5 percent/year, about 2.5 to about 5 percent/year, about 3 to about 5 percent/year, about 3.5 to about 5 percent/year, about 4 to about 5 percent/year, about 4.5 to about 5 percent/year, about 1 to about 1.5 percent/year, about 1.5 to about 2 percent/year, about 2 to about 2.5 percent/year, about 2.5 to about 3 percent/year, about 3 to about 3.5 percent/year, about 3.5 to about 4 percent/year, about 4 to about 4.5 percent/year, or about 4.5 to about 5 percent/year lower than the percent/year increase in total kidney volume of a subject not receiving the fixed-dose combination, or receiving only about 120 mg to about 240 mg bempedoic acid, or receiving only about 5 mg to about 90 mg tolvaptan.

In some embodiments, the subject receiving the fixed-dose combination has a composite incidence of clinical endpoints selected from worsening kidney function, kidney pain, hypertension, and albuminuria of about 5 to about 50 percent, about 10 to about 50 percent, about 15 to about 50 percent, about 20 to about 50 percent, about 25 to about 50 percent, about 30 to about 50 percent, about 35 to about 50 percent, about 40 to about 50 percent, about 45 to about 50 percent, about 5 to about 45 percent, about 5 to about 40 percent, about 5 to about 35 percent, about 5 to about 30 percent, about 5 to about 25 percent, about 5 to about 20 percent, about 5 to about 15 percent, about 5 to about 10 percent, about 15 to about 40 percent, about 20 to about 40 percent, about 25 to about 40 percent, about 30 to about 40 percent, about 35 to about 40 percent, about 15 to about 20 percent, about 20 to about 25 percent, about 25 to about 30 percent, about 30 to about 35 percent, or about 35 to about 40 percent lower than the composite incidence of clinical endpoints of a subject not receiving the fixed-dose combination, or receiving only about 30 mg to about 240 mg bempedoic acid, or receiving only about 5 mg to about 90 mg tolvaptan.

In some embodiments, the subject receiving the fixed-dose combination has a composite incidence of clinical endpoints selected from worsening kidney function, kidney pain, hypertension, and albuminuria of about 5 to about 50 percent, about 10 to about 50 percent, about 15 to about 50 percent, about 20 to about 50 percent, about 25 to about 50 percent, about 30 to about 50 percent, about 35 to about 50 percent, about 40 to about 50 percent, about 45 to about 50 percent, about 5 to about 45 percent, about 5 to about 40 percent, about 5 to about 35 percent, about 5 to about 30 percent, about 5 to about 25 percent, about 5 to about 20 percent, about 5 to about 15 percent, about 5 to about 10 percent, about 15 to about 40 percent, about 20 to about 40 percent, about 25 to about 40 percent, about 30 to about 40 percent, about 35 to about 40 percent, about 15 to about 20 percent, about 20 to about 25 percent, about 25 to about 30 percent, about 30 to about 35 percent, or about 35 to about 40 percent lower than the composite incidence of clinical endpoints of a subject not receiving the fixed-dose combination, or receiving only about 120 mg to about 240 mg bempedoic acid, or receiving only about 5 mg to about 90 mg tolvaptan.

In some embodiments, the subject receiving the fixed-dose combination has higher liver AMPK activity than a subject not receiving the fixed-dose combination, or receiving only about 5 mg to about 90 mg tolvaptan. In some embodiments, the subject receiving the fixed-dose combination has a higher degree of liver ACC phosphorylation than a subject not receiving the fixed-dose combination, or receiving only about 5 mg to about 90 mg tolvaptan. In some embodiments, the subject receiving the fixed-dose combination has a higher degree of peroxisome proliferator-activated receptor gamma coactivator 1-α (PGC1α) activity than a subject receiving only about 5 mg to about 90 mg tolvaptan.

In some embodiments, the fixed-dose combination is delivered orally. Examples of oral dosage forms include, but are not limited to a drench, a tablet, a capsule, a softgel capsule, a cachet, a pill, an emulsion, a lozenge, a solution, a suspension, a bolus, a powder, an elixir or syrup, a pastille, a mouthwash, a granule, or a paste for application to the tongue. In some embodiments, the pharmaceutical composition is formulated as a tablet.

In some embodiments, the fixed-dose combination comprises about 180 mg bempedoic acid. In some embodiments, the fixed-dose combination comprises about 5 mg, about 10 mg, about 15 mg, about 30 mg, about 45 mg, about 60 mg, or about 90 mg tolvaptan.

Also provided herein are methods of treating ADPKD in a subject in need thereof, the methods comprising administering to the subject an effective amount of bempedoic acid and tolvaptan. Also provided herein are methods of slowing the progression of ADPKD in a subject in need thereof, the methods comprising administering to the subject an effective amount of bempedoic acid and tolvaptan. Also provided herein are methods of preventing kidney failure, reducing total kidney volume, and/or slowing the progression of kidney dysfunction in a subject with ADPKD in need thereof, the methods comprising administering to the subject an effective amount of bempedoic acid and tolvaptan.

In some embodiments, the effective amount of bempedoic acid and tolvaptan comprises about 30 mg to about 240 mg bempedoic acid. In some embodiments, the effective amount of bempedoic acid and tolvaptan comprises about 120 mg to about 240 mg bempedoic acid. In certain embodiments, the effective amount of bempedoic acid and tolvaptan comprises about 180 mg bempedoic acid. In some embodiments, the effective amount of bempedoic acid and tolvaptan comprises about 5 mg, about 10 mg, about 15 mg, about 30 mg, about 45 mg, about 60 mg, or about 90 mg tolvaptan.

In some embodiments, the subject receiving the effective amount of bempedoic acid and tolvaptan has a lower total kidney weight to body weight ratio than a subject receiving a placebo, or receiving only bempedoic acid, or receiving only tolvaptan. In some embodiments, the subject receiving the effective amount of bempedoic acid and tolvaptan has a lower blood urea nitrogen level than a subject receiving a placebo, or receiving only bempedoic acid, or receiving only tolvaptan.

In some embodiments, the subject receiving the effective amount of bempedoic acid and tolvaptan has a total kidney weight to body weight ratio about 10 to about 140 percent, about 20 to about 140 percent, about 30 to about 140 percent, about 40 to about 140 percent, about 50 to about 140 percent, about 60 to about 140 percent, about 70 to about 140 percent, about 80 to about 140 percent, about 90 to about 140 percent, about 100 to about 140 percent, about 110 to about 140 percent, about 120 to about 140 percent, about 130 to about 140 percent, about 10 to about 80 percent, about 10 to about 70 percent, about 10 to about 60 percent, about 10 to about 50 percent, about 10 to about 40 percent, about 10 to about 30 percent, about 10 to about 20 percent, about 30 to about 70 percent, about 40 to about 70 percent, about 50 to about 70 percent, about 60 to about 70 percent, about 40 to about 60 percent, about 40 to about 50 percent, about 10 to about 20 percent, about 20 to about 30 percent, about 30 to about 40 percent, about 40 to about 50 percent, about 50 percent to about 60 percent, about 60 percent to about 70 percent, about 70 percent to about 80 percent, about 80 percent to about 90 percent, about 90 to about 100 percent, about 100 to about 110 percent, about 110 to about 120 percent, about 120 to about 130 percent, or about 130 percent to about 140 percent lower than the total kidney weight to body weight ratio of a subject receiving a placebo, or receiving only bempedoic acid, or receiving only tolvaptan.

In some embodiments, the subject receiving the effective amount of bempedoic acid and tolvaptan has a BUN level about 2 to about 15 mg/dL, about 3 to about 15 mg/dL, about 4 to about 15 mg/dL, about 5 to about 15 mg/dL, about 6 to about 15 mg/dL, about 7 to about 15 mg/dL, about 8 to about 15 mg/dL, about 9 to about 15 mg/dL, about 10 to about 15 mg/dL, about 11 to about 15 mg/dL, about 12 to about 15 mg/dL, about 13 to about 15 mg/dL, about 14 to about 15 mg/dL, about 2 to about 14 mg/dL, about 2 to about 13 mg/dL, about 2 to about 12 mg/dL, about 2 to about 11 mg/dL, about 2 to about 10 mg/dL, about 2 to about 9 mg/dL, about 2 to about 8 mg/dL, about 2 to about 7 mg/dL, about 2 to about 6 mg/dL, about 2 to about 5 mg/dL, about 2 to about 4 mg/dL, about 2 to about 3 mg/dL, about 3 to about 9 mg/dL, about 4 to about 9 mg/dL, about 5 to about 9 mg/dL, about 6 to about 9 mg/dL, about 7 to about 9 mg/dL, about 8 to about 9 mg/dL, about 3 to about 8 mg/dL, about 3 to about 7 mg/dL, about 3 to about 6 mg/dL, about 3 to about 5 mg/dL, or about 3 to about 4 mg/dL lower than the BUN of a subject receiving a placebo, or receiving only bempedoic acid, or receiving only tolvaptan.

In some embodiments, the subject receiving the effective amount of bempedoic acid and tolvaptan has a decrease in eGFR of about 0.5 to about 5 mL/min/1.73m²/year, about 1 to about 5 mL/min/1.73m²/year, about 1.5 to about 5 mL/min/1.73m²/year, about 2 to about 5 mL/min/1.73m²/year, about 2.5 to about 5 mL/min/1.73m²/year, about 3 to about 5 mL/min/1.73m²/year, about 3.5 to about 5 mL/min/1.73m²/year, about 4 to about 5 mL/min/1.73m²/year, about 4.5 to about 5 mL/min/1.73m²/year, about 0.5 to about 4.5 mL/min/1.73m²/year, about 0.5 to about 4 mL/min/1.73m²/year, about 0.5 to about 3.5 mL/min/1.73m²/year, about 0.5 to about 3 mL/min/1.73m²/year, about 0.5 to about 2.5 mL/min/1.73m²/year, about 0.5 to about 2 mL/min/1.73m²/year, about 0.5 to about 1.5 mL/min/1.73m²/year, about 0.5 to about 1 mL/min/1.73m²/year, about 1.7 to about 3.4 mL/min/1.73m²/year, about 1.9 to about 3.4 mL/min/1.73m²/year, about 2.1 to about 3.4 mL/min/1.73m²/year, about 2.3 to about 3.4 mL/min/1.73m²/year, about 2.5 to about 3.4 mL/min/1.73m²/year, about 2.7 to about 3.4 mL/min/1.73m²/year, about 2.9 to about 3.4 mL/min/1.73m²/year, about 3.1 to about 3.4 mL/min/1.73m²/year, about 3.3 to about 3.4 mL/min/1.73m²/year, about 1.7 to about 1.9 mL/min/1.73m²/year, about 1.9 to about 2.1 mL/min/1.73m²/year, about 2.1 to about 2.3 mL/min/1.73m²/year, about 2.3 to about 2.5 mL/min/1.73m²/year, about 2.5 to about 2.7 mL/min/1.73m²/year, about 2.7 to about 2.9 mL/min/1.73m²/year, about 2.9 to about 3.1 mL/min/1.73m²/year, about 3.1 to about 3.3 mL/min/1.73m²/year, or about 3.3 to about 3.4 mL/min/1.73m²/year lower than the decrease in eGFR per year of a subject receiving a placebo, or receiving only bempedoic acid, or receiving only tolvaptan.

In some embodiments, the subject receiving the effective amount of bempedoic acid and tolvaptan has an increase in total kidney volume of about 0 to about 7 percent/year, about 0.5 to about 7 percent/year, about 1 to about 7 percent/year, about 1.5 to about 7 percent/year, about 2 to about 7 percent/year, about 2.5 to about 7 percent/year, about 3 to about 7 percent/year, about 3.5 to about 7 percent/year, about 4 to about 7 percent/year, about 4.5 to about 7 percent/year, about 5 to about 7 percent/year, about 5.5 to about 7 percent/year, about 6 to about 7 percent/year, about 6.5 to about 7 percent/year, about 0 to about 6.5 percent/year, about 0 to about 6 percent/year, about 0 to about 5.5 percent/year, about 0 to about 5 percent/year, about 0 to about 4.5 percent/year, about 0 to about 4 percent/year, about 0 to about 3.5 percent/year, about 0 to about 3 percent/year, about 0 to about 2.5 percent/year, about 0 to about 2 percent/year, about 0 to about 1.5 percent/year, about 0 to about 1 percent/year, about 0 to about 0.5 percent/year, about 1 to about 5 percent/year, about 1.5 to about 5 percent/year, about 2 to about 5 percent/year, about 2.5 to about 5 percent/year, about 3 to about 5 percent/year, about 3.5 to about 5 percent/year, about 4 to about 5 percent/year, about 4.5 to about 5 percent/year, about 1 to about 1.5 percent/year, about 1.5 to about 2 percent/year, about 2 to about 2.5 percent/year, about 2.5 to about 3 percent/year, about 3 to about 3.5 percent/year, about 3.5 to about 4 percent/year, about 4 to about 4.5 percent/year, or about 4.5 to about 5 percent/year lower than the percent/year increase in total kidney volume of a subject receiving a placebo, or receiving only bempedoic acid, or receiving only tolvaptan.

In some embodiments, the subject receiving the effective amount of bempedoic acid and tolvaptan has a composite incidence of clinical endpoints selected from worsening kidney function, kidney pain, hypertension, and albuminuria of about 5 to about 50 percent, about 10 to about 50 percent, about 15 to about 50 percent, about 20 to about 50 percent, about 25 to about 50 percent, about 30 to about 50 percent, about 35 to about 50 percent, about 40 to about 50 percent, about 45 to about 50 percent, about 5 to about 45 percent, about 5 to about 40 percent, about 5 to about 35 percent, about 5 to about 30 percent, about 5 to about 25 percent, about 5 to about 20 percent, about 5 to about 15 percent, about 5 to about 10 percent, about 15 to about 40 percent, about 20 to about 40 percent, about 25 to about 40 percent, about 30 to about 40 percent, about 35 to about 40 percent, about 15 to about 20 percent, about 20 to about 25 percent, about 25 to about 30 percent, about 30 to about 35 percent, or about 35 to about 40 percent lower than the composite incidence of clinical endpoints of a subject receiving a placebo, or receiving only bempedoic acid, or receiving only tolvaptan.

In some embodiments, the subject receiving the effective amount of bempedoic acid and tolvaptan has higher liver AMPK activity than a subject receiving a placebo, or receiving only tolvaptan. In some embodiments, the subject receiving the effective amount of bempedoic acid and tolvaptan has a higher degree of liver ACC phosphorylation than a subject receiving a placebo, or receiving only tolvaptan. In some embodiments, the subject receiving the effective amount of bempedoic acid and tolvaptan has a higher degree of peroxisome proliferator-activated receptor gamma coactivator 1-α (PGC1α) activity than a subject receiving only tolvaptan.

In some embodiments, the effective amount of bempedoic acid and the effective amount of tolvaptan are delivered orally. Examples of oral dosage forms include, but are not limited to a drench, a tablet, a capsule, a softgel capsule, a cachet, a pill, an emulsion, a lozenge, a solution, a suspension, a bolus, a powder, an elixir or syrup, a pastille, a mouthwash, a granule, or a paste for application to the tongue. In some embodiments, the pharmaceutical composition is formulated as a tablet.

Also provided herein are methods of treating ADPKD in a subject in need thereof, the methods comprising administering to the subject an effective amount of bempedoic acid. Also provided herein are methods of slowing the progression of ADPKD in a subject in need thereof, the methods comprising administering to the subject an effective amount of bempedoic acid. Also provided herein are methods of preventing kidney failure, reducing total kidney volume, and/or slowing the progression of kidney dysfunction in a subject with ADPKD in need thereof, the methods comprising administering to the subject an effective amount of bempedoic acid.

In some embodiments, the effective amount of bempedoic acid is about 30 mg to about 240 mg. In some embodiments, the effective amount of bempedoic acid is about 120 mg to about 240 mg. In certain embodiments, the effective amount of bempedoic acid is about 180 mg.

In some embodiments, the subject receiving the effective amount of bempedoic acid has a lower total kidney weight to body weight ratio than a subject receiving a placebo, or has an equivalent or a lower total kidney weight to body weight ratio than a subject receiving tolvaptan therapy. In some embodiments, the subject receiving the effective amount of bempedoic acid has a lower blood urea nitrogen level than a subject receiving a placebo, or has an equivalent or a lower blood urea nitrogen level than a subject receiving tolvaptan therapy.

In some embodiments, the subject receiving the effective amount of bempedoic acid has a total kidney weight to body weight ratio about 10 to about 140 percent, about 20 to about 140 percent, about 30 to about 140 percent, about 40 to about 140 percent, about 50 to about 140 percent, about 60 to about 140 percent, about 70 to about 140 percent, about 80 to about 140 percent, about 90 to about 140 percent, about 100 to about 140 percent, about 110 to about 140 percent, about 120 to about 140 percent, about 130 to about 140 percent, about 10 to about 80 percent, about 10 to about 70 percent, about 10 to about 60 percent, about 10 to about 50 percent, about 10 to about 40 percent, about 10 to about 30 percent, about 10 to about 20 percent, about 30 to about 70 percent, about 40 to about 70 percent, about 50 to about 70 percent, about 60 to about 70 percent, about 40 to about 60 percent, about 40 to about 50 percent, about 10 to about 20 percent, about 20 to about 30 percent, about 30 to about 40 percent, about 40 to about 50 percent, about 50 percent to about 60 percent, about 60 percent to about 70 percent, about 70 percent to about 80 percent, about 80 percent to about 90 percent, about 90 to about 100 percent, about 100 to about 110 percent, about 110 to about 120 percent, about 120 to about 130 percent, or about 130 percent to about 140 percent lower than the total kidney weight to body weight ratio of a subject receiving a placebo, or has an equivalent or a lower total kidney weight to body weight ratio than the total kidney weight to body weight ratio of a subject receiving tolvaptan therapy.

In some embodiments, the subject receiving the effective amount of bempedoic acid has a BUN level about 2 to about 15 mg/dL, about 3 to about 15 mg/dL, about 4 to about 15 mg/dL, about 5 to about 15 mg/dL, about 6 to about 15 mg/dL, about 7 to about 15 mg/dL, about 8 to about 15 mg/dL, about 9 to about 15 mg/dL, about 10 to about 15 mg/dL, about 11 to about 15 mg/dL, about 12 to about 15 mg/dL, about 13 to about 15 mg/dL, about 14 to about 15 mg/dL, about 2 to about 14 mg/dL, about 2 to about 13 mg/dL, about 2 to about 12 mg/dL, about 2 to about 11 mg/dL, about 2 to about 10 mg/dL, about 2 to about 9 mg/dL, about 2 to about 8 mg/dL, about 2 to about 7 mg/dL, about 2 to about 6 mg/dL, about 2 to about 5 mg/dL, about 2 to about 4 mg/dL, about 2 to about 3 mg/dL, about 3 to about 9 mg/dL, about 4 to about 9 mg/dL, about 5 to about 9 mg/dL, about 6 to about 9 mg/dL, about 7 to about 9 mg/dL, about 8 to about 9 mg/dL, about 3 to about 8 mg/dL, about 3 to about 7 mg/dL, about 3 to about 6 mg/dL, about 3 to about 5 mg/dL, or about 3 to about 4 mg/dL lower than the BUN of a subject receiving a placebo, or has an equivalent or a lower BUN than the BUN of a subject receiving tolvaptan therapy.

In some embodiments, the subject receiving the effective amount of bempedoic acid has a decrease in eGFR of about 0.5 to about 5 mL/min/1.73m²/year, about 1 to about 5 mL/min/1.73m²/year, about 1.5 to about 5 mL/min/1.73m²/year, about 2 to about 5 mL/min/1.73m²/year, about 2.5 to about 5 mL/min/1.73m²/year, about 3 to about 5 mL/min/1.73m²/year, about 3.5 to about 5 mL/min/1.73m²/year, about 4 to about 5 mL/min/1.73m²/year, about 4.5 to about 5 mL/min/1.73m²/year, about 0.5 to about 4.5 mL/min/1.73m²/year, about 0.5 to about 4 mL/min/1.73m²/year, about 0.5 to about 3.5 mL/min/1.73m²/year, about 0.5 to about 3 mL/min/1.73m²/year, about 0.5 to about 2.5 mL/min/1.73m²/year, about 0.5 to about 2 mL/min/1.73m²/year, about 0.5 to about 1.5 mL/min/1.73m²/year, about 0.5 to about 1 mL/min/1.73m²/year, about 1.7 to about 3.4 mL/min/1.73m²/year, about 1.9 to about 3.4 mL/min/1.73m²/year, about 2.1 to about 3.4 mL/min/1.73m²/year, about 2.3 to about 3.4 mL/min/1.73m²/year, about 2.5 to about 3.4 mL/min/1.73m²/year, about 2.7 to about 3.4 mL/min/1.73m²/year, about 2.9 to about 3.4 mL/min/1.73m²/year, about 3.1 to about 3.4 mL/min/1.73m²/year, about 3.3 to about 3.4 mL/min/1.73m²/year, about 1.7 to about 1.9 mL/min/1.73m²/year, about 1.9 to about 2.1 mL/min/1.73m²/year, about 2.1 to about 2.3 mL/min/1.73m²/year, about 2.3 to about 2.5 mL/min/1.73m²/year, about 2.5 to about 2.7 mL/min/1.73m²/year, about 2.7 to about 2.9 mL/min/1.73m²/year, about 2.9 to about 3.1 mL/min/1.73m²/year, about 3.1 to about 3.3 mL/min/1.73m²/year, or about 3.3 to about 3.4 mL/min/1.73m²/year lower than the decrease in eGFR per year of a subject receiving a placebo, or has an equivalent or a lower decrease in eGFR per year than the decrease in eGFR per year of a subject receiving tolvaptan therapy.

In some embodiments, the subject receiving the effective amount of bempedoic acid has an increase in total kidney volume of about 0 to about 7 percent/year, about 0.5 to about 7 percent/year, about 1 to about 7 percent/year, about 1.5 to about 7 percent/year, about 2 to about 7 percent/year, about 2.5 to about 7 percent/year, about 3 to about 7 percent/year, about 3.5 to about 7 percent/year, about 4 to about 7 percent/year, about 4.5 to about 7 percent/year, about 5 to about 7 percent/year, about 5.5 to about 7 percent/year, about 6 to about 7 percent/year, about 6.5 to about 7 percent/year, about 0 to about 6.5 percent/year, about 0 to about 6 percent/year, about 0 to about 5.5 percent/year, about 0 to about 5 percent/year, about 0 to about 4.5 percent/year, about 0 to about 4 percent/year, about 0 to about 3.5 percent/year, about 0 to about 3 percent/year, about 0 to about 2.5 percent/year, about 0 to about 2 percent/year, about 0 to about 1.5 percent/year, about 0 to about 1 percent/year, about 0 to about 0.5 percent/year, about 1 to about 5 percent/year, about 1.5 to about 5 percent/year, about 2 to about 5 percent/year, about 2.5 to about 5 percent/year, about 3 to about 5 percent/year, about 3.5 to about 5 percent/year, about 4 to about 5 percent/year, about 4.5 to about 5 percent/year, about 1 to about 1.5 percent/year, about 1.5 to about 2 percent/year, about 2 to about 2.5 percent/year, about 2.5 to about 3 percent/year, about 3 to about 3.5 percent/year, about 3.5 to about 4 percent/year, about 4 to about 4.5 percent/year, or about 4.5 to about 5 percent/year lower than the percent/year increase in total kidney volume of a subject receiving a placebo, or has an equivalent or a lower percent/year increase in total kidney volume than the percent/year increase in total kidney volume of a subject receiving only tolvaptan.

In some embodiments, the subject receiving the effective amount of bempedoic acid has a composite incidence of clinical endpoints selected from worsening kidney function, kidney pain, hypertension, and albuminuria of about 5 to about 50 percent, about 10 to about 50 percent, about 15 to about 50 percent, about 20 to about 50 percent, about 25 to about 50 percent, about 30 to about 50 percent, about 35 to about 50 percent, about 40 to about 50 percent, about 45 to about 50 percent, about 5 to about 45 percent, about 5 to about 40 percent, about 5 to about 35 percent, about 5 to about 30 percent, about 5 to about 25 percent, about 5 to about 20 percent, about 5 to about 15 percent, about 5 to about 10 percent, about 15 to about 40 percent, about 20 to about 40 percent, about 25 to about 40 percent, about 30 to about 40 percent, about 35 to about 40 percent, about 15 to about 20 percent, about 20 to about 25 percent, about 25 to about 30 percent, about 30 to about 35 percent, or about 35 to about 40 percent lower than the composite incidence of clinical endpoints of a subject receiving a placebo, or has an equivalent or a lower incidence of clinical endpoints selected from worsening kidney function, kidney pain, hypertension, and albuminuria than a subject receiving tolvaptan therapy.

In some embodiments, the effective amount of bempedoic acid is delivered orally. Examples of oral dosage forms include, but are not limited to a drench, a tablet, a capsule, a softgel capsule, a cachet, a pill, an emulsion, a lozenge, a solution, a suspension, a bolus, a powder, an elixir or syrup, a pastille, a mouthwash, a granule, or a paste for application to the tongue. In some embodiments, the pharmaceutical composition is formulated as a tablet.

Also provided herein are methods of treating dyslipidemia in a subject in need thereof, the methods generally comprising administering to the subject an effective amount of tolvaptan and an effective amount of bempedoic acid. In some embodiments, the method lowers LDL-C in the blood or serum of a subject. In some embodiments, the method lowers systolic and/or diastolic blood pressure in a subject. In some embodiments, the method lowers hsCRP in the blood or serum of a subject. In some embodiments, the method lowers the body weight of the subject. In some embodiments, the method reduces the risk of atherosclerotic cardiovascular disease in a subject.

In some embodiments, tolvaptan is delivered orally. In some embodiments, the bempedoic acid and tolvaptan are formulated as an oral dosage form. Examples of oral dosage forms include, but are not limited to a drench, a tablet, a capsule, a softgel capsule, a cachet, a pill, an emulsion, a lozenge, a solution, a suspension, a bolus, a powder, an elixir or syrup, a pastille, a mouthwash, a granule, or a paste for application to the tongue. In some embodiments, the pharmaceutical composition is formulated as a tablet.

In some embodiments, the methods disclosed herein lower the level of apo B in blood or serum of the subject below that of a subject not receiving the tolvaptan and bempedoic acid combination. In some embodiments, the methods disclosed herein lower the level of non-high-density lipoprotein cholesterol in blood or serum of the subject below that of a subject not receiving the tolvaptan and bempedoic acid combination. In some embodiments, the methods disclosed herein lower the level of triglycerides in blood or serum of the subject below that of a subject not receiving the tolvaptan and bempedoic acid combination. In some embodiments, the methods disclosed herein lower the LDL particle number in blood or serum of the subject below that of a subject not receiving the tolvaptan and bempedoic acid combination. In some embodiments, the methods disclosed herein decrease the size of VLDL particles in blood or serum of the subject below that of a subject not receiving the tolvaptan and bempedoic acid combination. In some embodiments, the methods disclosed herein decrease the number of VLDL particles in blood or serum of the subject below that of a subject not receiving tolvaptan and bempedoic acid combination. In some embodiments, the methods disclosed herein increase the level of apo A1 in blood or serum of the subject above that of a subject not receiving the tolvaptan and bempedoic acid combination. In some embodiments, the methods disclosed herein do not change the level of apo A1 in blood or serum of the subject compared to that of a subject not receiving the tolvaptan and bempedoic acid combination. In some embodiments, the methods disclosed herein decrease the level of apo A1 in blood or serum of the subject below that of a subject not receiving the tolvaptan and bempedoic acid combination.

In certain embodiments, the methods disclosed herein comprise administering to a subject the combined therapeutic agents (e.g., bempedoic acid and tolvaptan) as a single dosage form. In certain embodiments, the methods disclosed herein comprise administering to a subject the therapeutic agents (e.g., bempedoic acid and a tolvaptan) as separate dosage forms.

In some embodiments, the subject is a human.

### EXAMPLES

Below are examples of specific embodiments for carrying out the present disclosure. The examples are offered for illustrative purposes only, and are not intended to limit the scope of the present disclosure in any way.

Any terms not directly defined herein shall be understood to have the meanings commonly associated with them as understood within the art of the disclosure. Certain terms are discussed herein to provide additional guidance to the practitioner in describing the compositions, devices, methods and the like of aspects of the disclosure, and how to make or use them. It will be appreciated that the same thing may be said in more than one way. Consequently, alternative language and synonyms may be used for any one or more of the terms discussed herein. No significance is to be placed upon whether or not a term is elaborated or discussed herein. Some synonyms or substitutable methods, materials and the like are provided. Recital of one or a few synonyms or equivalents does not exclude use of other synonyms or equivalents, unless it is explicitly stated. Use of examples, including examples of terms, is for illustrative purposes only and does not limit the scope of the appended claims.

### Example 1 - Immunoblot Assays of FATP2 and ACLY Protein Expression in Various Mouse Tissues and Kidney Cell Lines

The example describes immunoblot assays to assess the FATP2 and ACLY protein expression in mouse liver, lung, kidney, spleen, and muscle tissues, as well as proximal tubule- and inner medullary collecting duct-derived mouse epithelial cells.

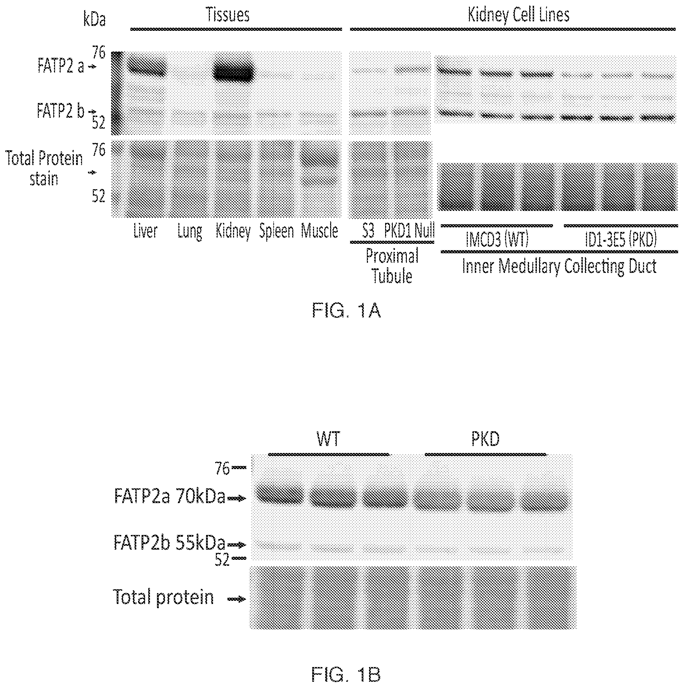

FATP2 (ACSVL1; SLC27A2) protein expression was observed in immunoblots of homogenates of different mouse tissues and kidney cell lines. Immunoblotting of various mouse tissues (**FIG. 1A**, left) revealed expression of two distinct FATP2 isoforms. The short splice variant (FATP2b at ~55 kDa), which lacks the protein domain required for conversion of bempedoic acid to its active metabolite bempedoic acid-CoA, was expressed in all tissues tested. However, the long form (FATP2a at ~70 kDa) had significant expression only in liver and kidney tissue, thus providing specificity of ETC-1002 pro-drug conversion to its active form only in these tissues. The FATP2a and FATP2b isoforms were expressed in immortalized mouse kidney epithelial cells that were derived from both proximal tubule and inner medullary collecting duct, both with or without knockout of the Pkd1 gene (**FIG. 1A**, right).

Similar protein expression levels of both isoforms of FATP2 were also found in both wild-type (WT) and ADPKD mutant (Pkd1RC/RC) mouse kidney tissue (**FIG. 1B**)).

ACLY protein correlated with disease severity in the hypomorphic *Pkd1*^{*RC*/*RC*} ADPKD mouse model. ACLY protein expression and activity was increased in Pkd1-/- kidney epithelial cells as compared to controls in vitro.

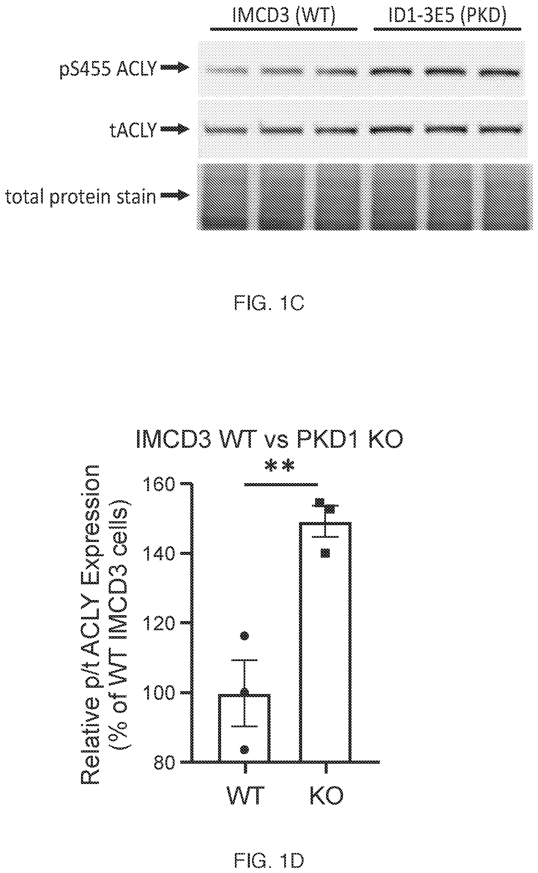

Immunoblotting of inner medullary collecting duct (IMCD)-derived mouse epithelial cells for expression of t ACLY (total ACLY) and pS455 ACLY (**FIG. 1C**) showed increased expression of activated (pS455) ACLY relative to t ACLY in Pkd1-null (ID1-3E5) cells as compared with wild-type (WT) (IMCD3) cells (densitometric quantitation revealed a significant ~50% increase in the pS455-ACLY/t-ACLY ratio in the ID1-3E5 cells (p < 0.05; unpaired t-test) (**FIG. 1D**)). A total protein stain shows comparable loading across the different lanes. Cells were pretreated with or without (+ or -, as indicated) a novel small molecule ACLY inhibitor prior to lysis and immunoblotting.

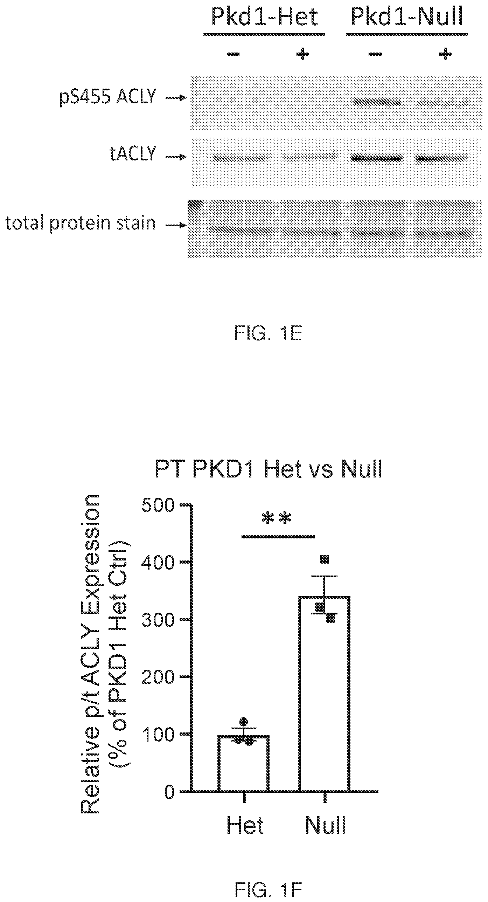

Immunoblotting of proximal tubule (PT)-derived mouse epithelial cells shows greater protein expression of total ACLY (t ACLY) and activated ACLY (**FIG. 1E**), as detected using a phospho-specific antibody directed against Ser455, a known AKT phosphorylation and activating site (pS455 ACLY), in cells with *Pkd1* expression knocked out at both alleles as compared with cells that are heterozygous for *Pkd1* deletion (densitometric quantitation revealed a significant ~250% increase in the pS455-ACLY/t-ACLY ratio in the ID1-3E5 cells (p < 0.05; unpaired t-test) (**FIG. 1F**).

### Example 2 - Growth Inhibition Studies of ACLY Inhibitors Bempedoic Acid (ETC-1002) and SB-204990 in Pkd1-/- Kidney Epithelial Cells in 3D Cultures

The example describes cell growth inhibition studies in 3D cultures of kidney epithelial cells using either of ACLY inhibitors bempedoic acid or SB-204990. SB-204990 is represented by the structure of Formula (III):

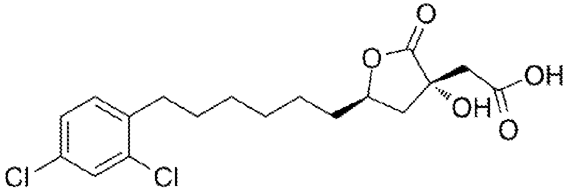

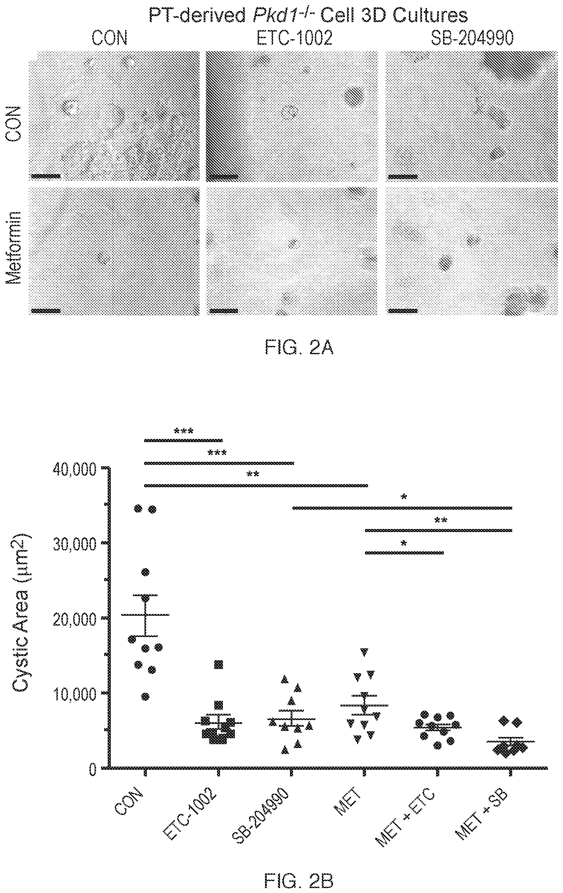

ACLY inhibitors inhibited cyst growth of proximal tubule (PT)-derived Pkd1-/- kidney epithelial cells in 3D cultures both alone. Cells were grown in Matrigel 12 days and treated with 10 µM forskolin + 100 µM IBMX for 11 days, and then treated with either vehicle (DMSO; CON), 500 µM metformin, 100 µM ETC-1002 (bempedoic acid) or 30 µM SB-204990 either alone or in combination as shown for the last 3 days (**FIG. 2A**). Summary data revealed that each of the treatments significantly reduced cyst area relative to control. Error bars indicate 95% confidence interval (CI) (*P < 0.05, **P < 0.01, ***P < 0.001 for the indicated comparisons by one-way ANOVA and Tukey's multiple comparison test) (**FIG. 2B**).

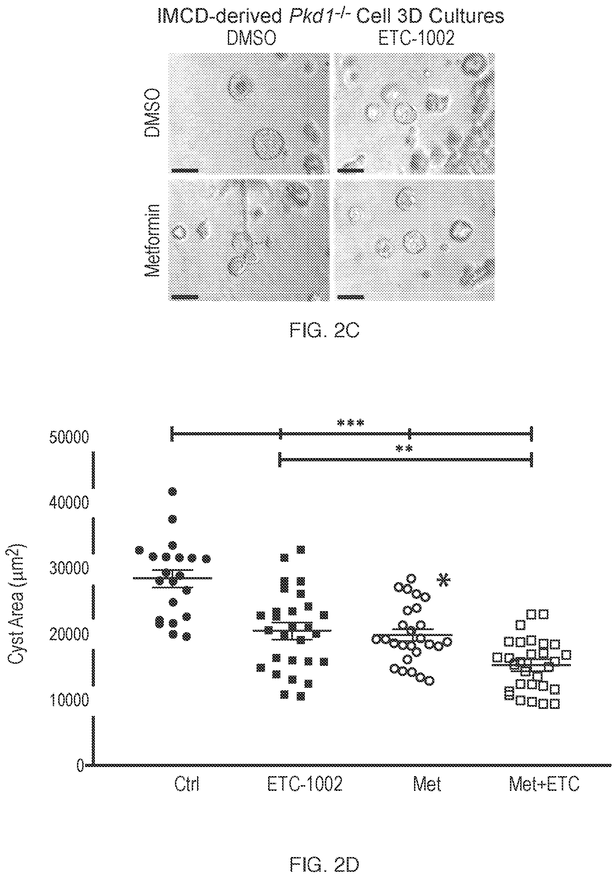

ETC-1002 inhibited cyst growth of inner medullary collecting duct (IMCD)-derived Pkd1-/- kidney epithelial cells in 3D cultures. Cells were grown in Matrigel for 6 days and treated with 10 µM forskolin + 100 µM IBMX ± 500 µM metformin and/or 100 µM of ETC-1002 (bempedoic acid) for the last 3 days alone or in combination as shown (**FIG. 2C**). Summary data revealed that each of the treatments alone significantly reduced cyst area relative to control. Error bars indicate 95% CI (*P < 0.05, **P < 0.01, ***P < 0.001 for the indicated comparisons by one-way ANOVA and Tukey's multiple comparison test) (**FIG. 2D**).

### Example 3 - Studies Examining the Effects of Bempedoic Acid and Tolvaptan Alone and in Combination in a Rapidly Developing Early Onset ADPKD Mouse Model

The example describes characterization of the early onset, rapidly developing ADPKD mouse model Pkd1fl/fl;Pax8-rtTA;Tet-O-Cre homozygous in the C57BL/6 background.

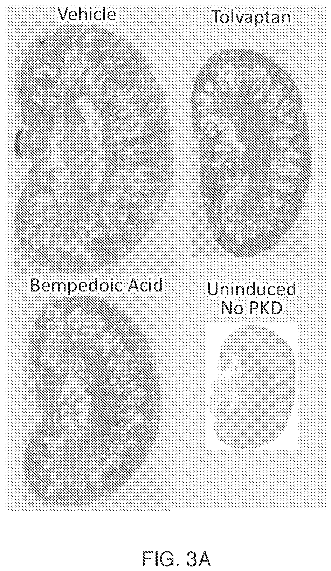

Doxycycline was administered intraperitoneally (50 mg/kg/day) on postnatal days 10 and 11 (P10 and P11) to induce Pkd1 deletion and rapidly progressive PKD in the Cre+ mice. Mice were treated with the various drugs and combinations or vehicle by oral gavage daily from P12-P21 and were then sacrificed at P22 of age to harvest kidneys for histology, weight measurements and blood urea nitrogen (BUN) measurements. Treatments were vehicle, tolvaptan 30 mg/kg/d, tolvaptan 100 mg/kg/d, bempedoic acid 30 mg/kg/d, tolvaptan 30 mg/kg/d + bempedoic acid 30 mg/kg/d, and tolvaptan 100 mg/kg/d + bempedoic acid 30 mg/kg/d; also included was an uninduced, untreated group without PKD. Each treatment group consisted of 3-6 mice. Representative H&E (hematoxylin and eosin) stains of kidney sections from Cre+ mice at P22 demonstrate a rapid increase in kidney size over time (vehicle vs uninduced). Administration of tolvaptan or ETC-1002 (bempedoic acid) each showed a marked reduction in kidney size when compared to vehicle control (**FIG. 3A**).

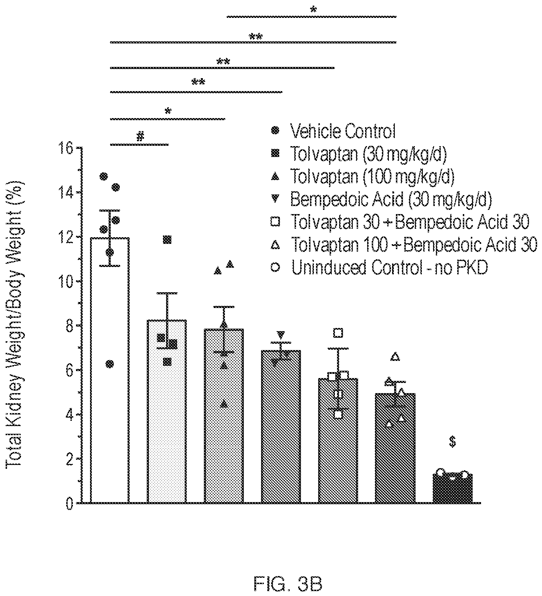

The total kidney weight to body weight ratios were dramatically increased at the time of euthanasia in the Cre+ mice relative to Cre- control mice. Administration of tolvaptan (30 mg/kg/d) or (100 mg/kg/d) each reduced total kidney weight to body weight ratio. Bempedoic acid (30/mg/kg/d) also significantly reduced total kidney weight to body weight ratio to a similar level when administered alone. Total kidney weight to body weight was further reduced when combined with tolvaptan (30 or 100 mg/kg/d), such that the combination was lower than either treatment alone (**FIG. 3B**). Bempedoic acid (30 mg/kg/d) reduced total kidney weight to body weight ratio relative to vehicle (6.9 ± 0.4% vs. 11.9 ± 1.2%; *P* <0.01, n = 3-6, unpaired t-test). Similarly, tolvaptan (100 mg/kg/d) reduced total kidney weight to body weight ratio relative to vehicle (7.8 ± 1.0%; *P* <0.05, n = 6). Addition of bempedoic acid to tolvaptan caused a further reduction in total kidney weight to body weight ratio relative to tolvaptan alone (4.9 ± 0.6%; *P <0.05*).

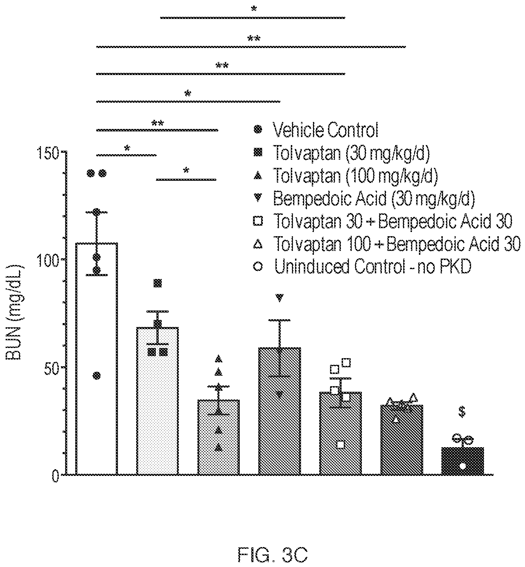

BUN was also dramatically increased at the time of euthanasia in the Cre+ mice relative to Cre- control mice. Administration of tolvaptan (30 mg/kg/d) or (100 mg/kg/d) each reduced BUN in a dose-dependent manner. Bempedoic acid (30 mg/kg/d) also significantly reduced BUN to a similar level as tolvaptan when administered alone, which was further reduced when combined with tolvaptan (30 or 100 mg/kg/d), such that the combination lowered BUN more than either treatment alone (**FIG. 3C**). Bempedoic acid (30 mg/kg/d) reduced BUN relative to vehicle (59 ± 13 vs. 107 ± 14 mg/dL; *P* <0.05). Tolvaptan treatment also dose-dependently decreased BUN relative to vehicle at 30 mg/kg/d (68 ± 8; *P* <0.05) and 100 mg/kg/d (35 ± 7; *P* <0.01). Addition of bempedoic to tolvaptan at 30 mg/kg/d caused a further significant reduction in BUN (38 ± 7; *P* <0.05).

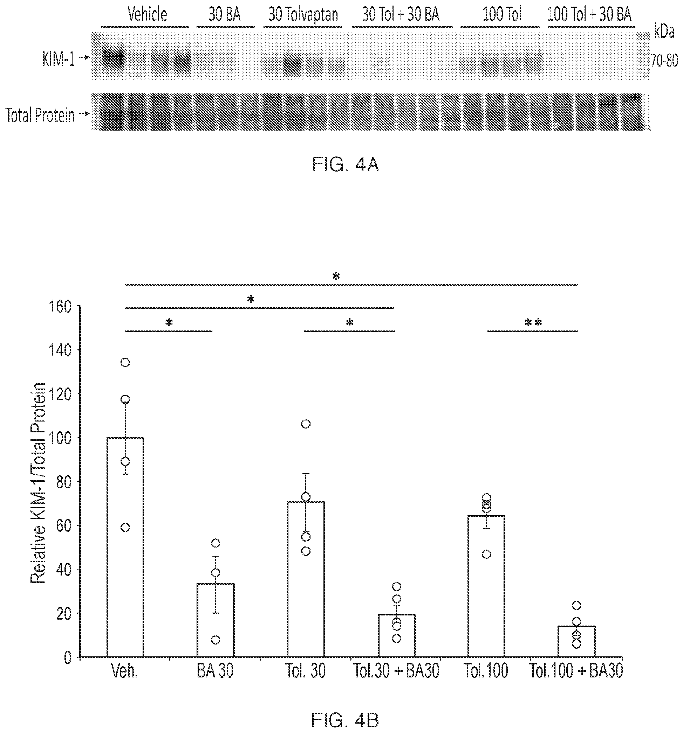

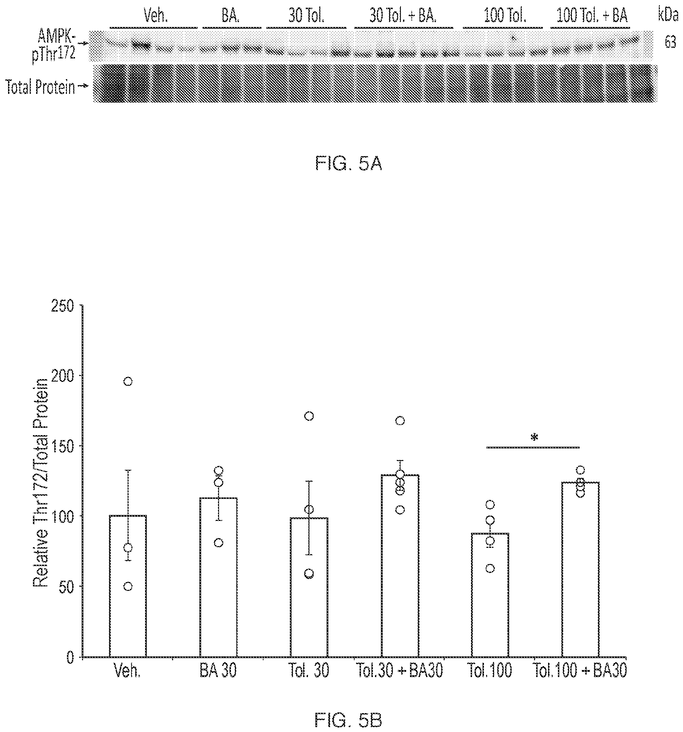

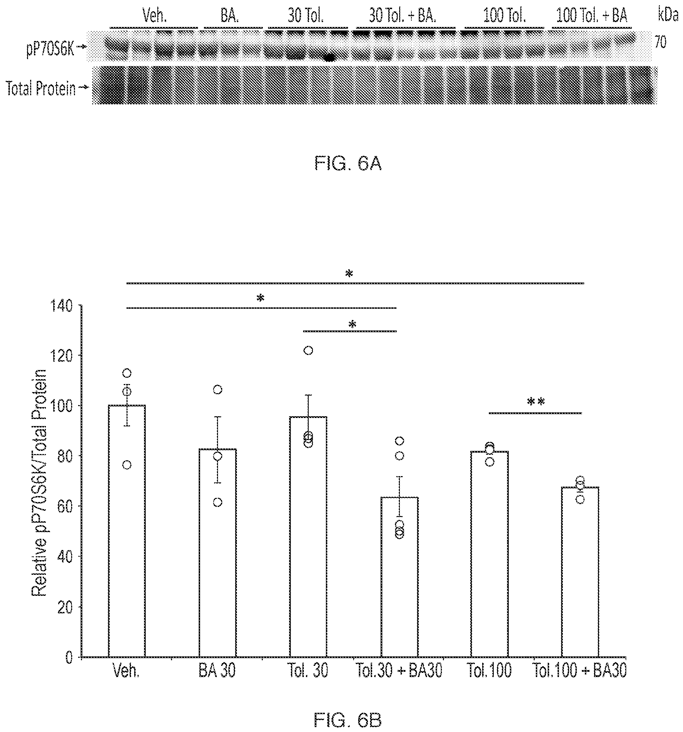

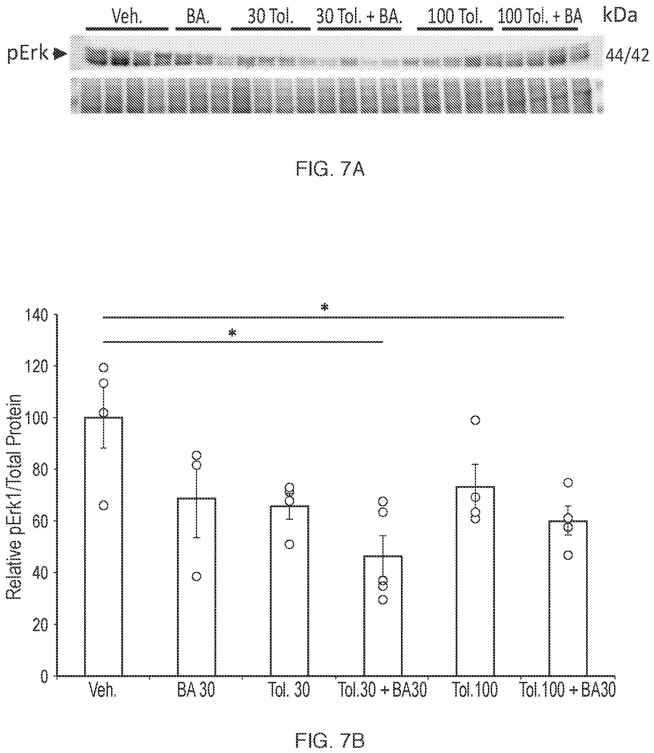

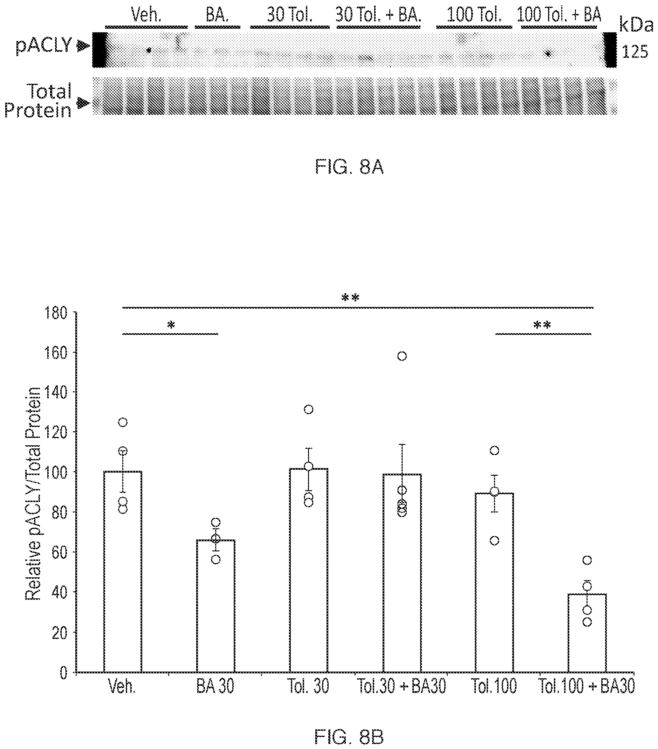

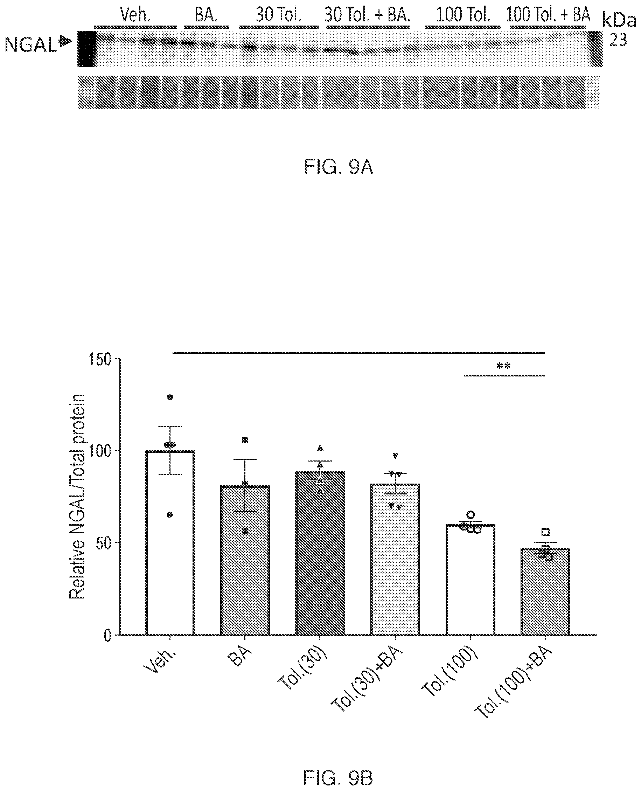

At the end of the study, kidney sample lysates were prepared in buffer and separated using SDS-PAGE. Separated proteins were then electrophoretically transferred to membranes. Nonspecific binding was blocked, and membranes were probed with antibodies against phosphorylated (Thr172)-AMPK, phosphorylated P70S6K, phosphorylated ERK, phosphorylated ACLY, total KIM-1, and total neutrophil gelatinase-associated lipocalin (NGAL). Membranes were incubated with appropriate detection antibodies and relative signal intensity determined. Protein levels were normalized to total protein levels. Bempedoic acid treatment reduced ACLY (**FIG. 8**) and stimulated AMPK (**FIG. 5A/5B**) activity. Bempedoic acid also inhibited the mTOR (**FIG. 6A/6B**), ERK (**FIG. 7**), and NGAL (**FIG. 9A/9B**) signaling pathways, which are upregulated in ADPKD. Finally, bempedoic acid also dramatically reduced KIM-1 expression, a marker of kidney injury, by ≥70% (**FIG. 4A/4B**), and, to a lesser extent, neutrophil gelatinase-associate lipocalin (NGAL). These effects of bempedoic acid occurred both alone and in combination with tolvaptan therapy.

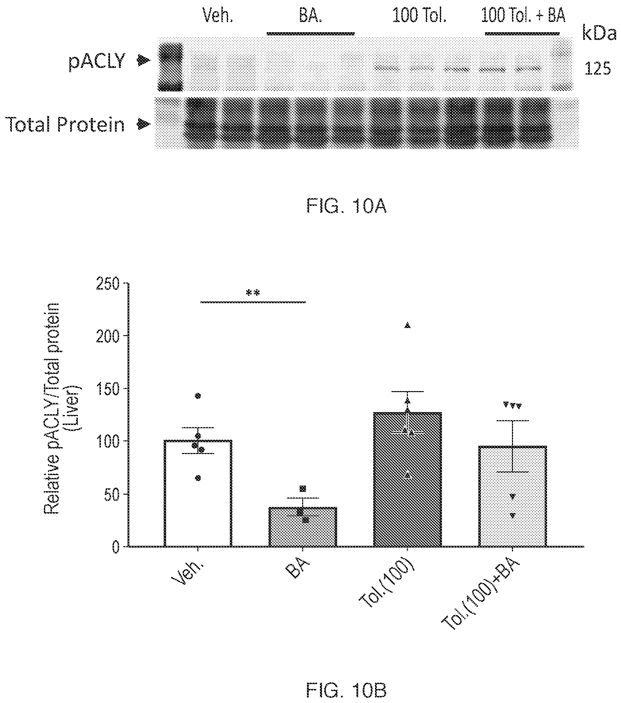

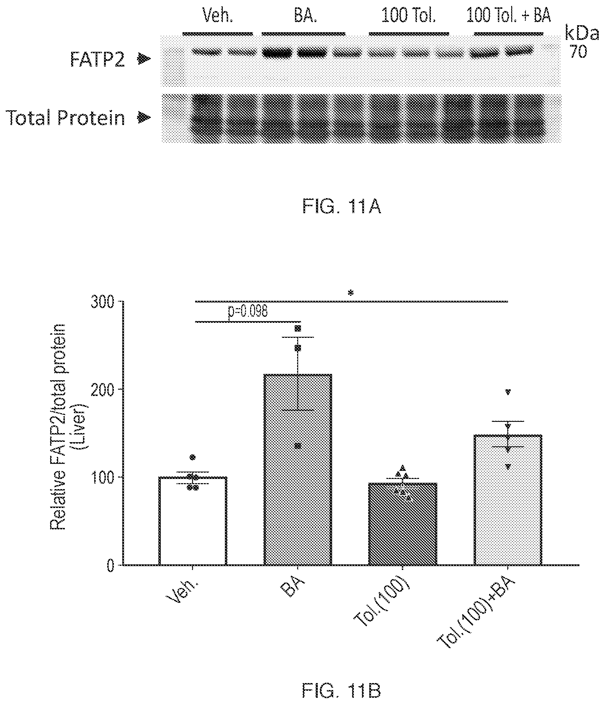

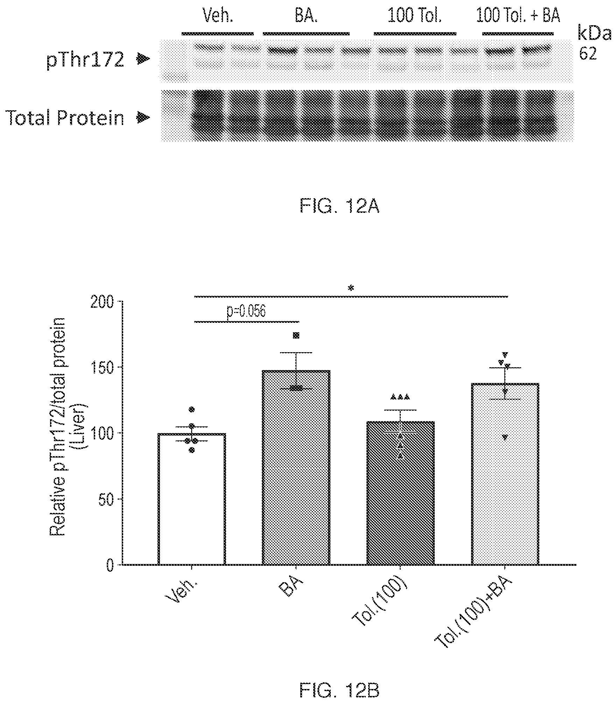

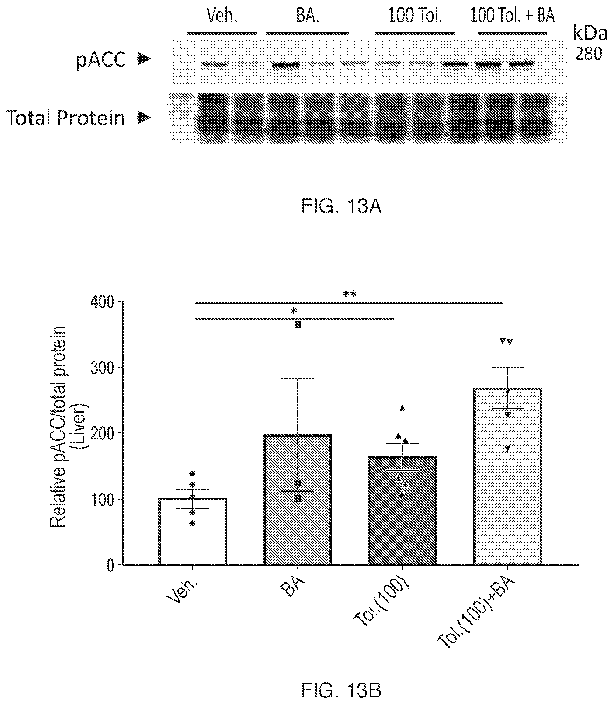

Liver sample lysates were prepared in buffer and separated using SDS-PAGE. Separated proteins were then electrophoretically transferred to membranes. Nonspecific binding was blocked and membranes were probed with antibodies against pACLY, pThr172AMPKα, PGC1alpha, FATP2, pACC, and cleaved Cas3. Membranes were incubated with appropriate detection antibodies and relative signal intensity determined. Target protein levels were normalized to total protein levels. Bempedoic acid inhibited pACLY (activation marker) (**FIG. 10A/10B**) and increased FATP2 expression (**FIG. 11A/11B**) both alone and in combination with tolvaptan. Bempedoic acid also increased AMPK activity (**FIG. 12A/12B**), and promoted phosphorylation of conical AMPK target ACC in liver tissue (**FIG. 13A/13B**).

### Example 4 - Studies Examining the Effects of Bempedoic Acid in a Slowly Developing Late Onset ADPKD Mouse Model

The example describes characterization of the late onset, slowly developing ADPKD mouse model Pkd1fl/fl;Pax8-rtTA;Tet-On-Cre when treated with bempedoic acid.

Doxycycline was administered intraperitoneally (50 mg/kg/day) on postnatal days 27-29 (P27-P29) and again on P43 and P57 to induce later Pkd1 knockout and slowly progressive PKD in the Cre+ mice. Mice were then sacrificed at P28, P60, and P120 for measurements of total kidney weight to body weight ratios. The ratio was dramatically increased in male control mice at P60, and both male and female control mice at P120 compared to P28 Cre- control mice. Treatment with bempedoic acid (30 mg/kg/d, mixed with food, beginning on P30 and continuing through P60 or P120) significantly reduced total kidney weight to body weight ratio at P60 in male mice, a trend that was also observed at P120 in both male and female mice (**FIG. 9**).

## Claims

1. A fixed-dose combination comprising bempedoic acid and tolvaptan.

2. The fixed-dose combination of claim 1, wherein the fixed-dose combination comprises about 30 mg to about 240 mg bempedoic acid.

3. The fixed-dose combination of claim 1 or 2, wherein the fixed-dose combination comprises about 5 mg to about 120 mg tolvaptan.

4. The fixed-dose combination of any one of claims 1-3 wherein the fixed-dose combination comprises about 180 mg bempedoic acid.

5. A pharmaceutical composition comprising:
bempedoic acid;
tolvaptan; and
one or more pharmaceutically acceptable excipients.

6. The pharmaceutical composition of claim 5, wherein the pharmaceutical composition comprises about 30 mg to about 240 mg bempedoic acid.

7. The pharmaceutical composition of claim 5 or 6, wherein the pharmaceutical composition comprises about 5 mg to about 120 mg tolvaptan.

8. The pharmaceutical composition of any one of claims 5-7, wherein the pharmaceutical composition comprises about 180 mg bempedoic acid.

9. The fixed-dose combination of any one of claims 1-4 or the pharmaceutical composition of any one of claim 5-8, wherein the fixed-dose combination or the pharmaceutical composition is formulated for oral delivery as an oral solid dosage form.

10. An effective amount of bempedoic acid for use in treating autosomal dominant polycystic kidney disease (ADPKD) or slowing the progression of ADPKD in a human receiving tolvaptan therapy or preventing kidney failure in a human with ADPKD receiving tolvaptan therapy.

11. Bempedoic acid for use according to claim 10, wherein the effective amount of bempedoic acid is about 30 mg to about 240 mg.

12. The fixed-dose combination of any one of claims 1-4 and 9, or the pharmaceutical composition of any one of claims 5-9 for use in treating autosomal dominant polycystic kidney disease (ADPKD) or slowing the progression of ADPKD in a human in need thereof or preventing kidney failure in a human with ADPKD.

13. An effective amount of bempedoic acid for use in treating autosomal dominant polycystic kidney disease (ADPKD) or slowing the progression of ADPKD in a human in need thereof or preventing kidney failure in a human with ADPKD.

14. Bempedoic acid for use according to claim 13, wherein the effective amount of bempedoic acid is about 30 mg to about 240 mg.

15. Bempedoic acid for use according to claim 13 or 14, said use further comprising administering to the subject an effective amount of tolvaptan.

## Patentansprüche

1. Fixdosiskombination, die Bempedoinsäure und Tolvaptan umfasst.

2. Fixdosiskombination nach Anspruch 1, wobei die Fixdosiskombination etwa 30 mg bis etwa 240 mg Bempedoinsäure umfasst.

3. Fixdosiskombination nach Anspruch 1 oder 2, wobei die Fixdosiskombination etwa 5 mg bis etwa 120 mg Tolvaptan umfasst.

4. Fixdosiskombination nach einem der Ansprüche 1 bis 3, wobei die Fixdosiskombination etwa 180 mg Bempedoinsäure umfasst.

5. Pharmazeutische Zusammensetzung, die Folgendes umfasst:
Bempedoinsäure;
Tolvaptan; und
einen oder mehrere pharmazeutisch annehmbare Hilfsstoffe.

6. Pharmazeutische Zusammensetzung nach Anspruch 5, wobei die pharmazeutische Zusammensetzung etwa 30 mg bis etwa 240 mg Bempedoinsäure umfasst.

7. Pharmazeutische Zusammensetzung nach Anspruch 5 oder 6, wobei die pharmazeutische Zusammensetzung etwa 5 mg bis etwa 120 mg Tolvaptan umfasst.

8. Pharmazeutische Zusammensetzung nach einem der Ansprüche 5 bis 7, wobei die pharmazeutische Zusammensetzung etwa 180 mg Bempedoinsäure umfasst.

9. Fixdosiskombination nach einem der Ansprüche 1 bis 4 oder pharmazeutische Zusammensetzung nach einem der Ansprüche 5 bis 8, wobei die Fixdosiskombination oder die pharmazeutische Zusammensetzung zur oralen Gabe als orale feste Dosierungsform formuliert ist.

10. Wirksame Menge von Bempedoinsäure zur Verwendung bei der Behandlung einer autosomal-dominanten polyzystischen Nierenkrankheit (ADPKD) oder Verlangsamung des Fortschreitens von ADPKD bei einem Menschen, der eine Tolvaptantherapie erhält, oder bei der Prävention von Nierenversagen bei einem Menschen mit ADPKD, der eine Tolvaptantherapie erhält.

11. Bempedoinsäure zur Verwendung nach Anspruch 10, wobei die wirksame Menge von Bempedoinsäure etwa 30 mg bis etwa 240 mg beträgt.

12. Fixdosiskombination nach einem der Ansprüche 1 bis 4 und 9 oder pharmazeutische Zusammensetzung nach einem der Ansprüche 5 bis 9 zur Verwendung bei der Behandlung einer autosomal-dominanten polyzystischen Nierenkrankheit (ADPKD) oder Verlangsamung des Fortschreitens von ADPKD bei einem bedürftigen Menschen oder bei der Prävention von Nierenversagen bei einem Menschen mit ADPKD.

13. Wirksame Menge von Bempedoinsäure zur Verwendung bei der Behandlung einer autosomal-dominanten polyzystischen Nierenkrankheit (ADPKD) oder Verlangsamung des Fortschreitens von ADPKD bei einem bedürftigen Menschen oder bei der Prävention von Nierenversagen bei einem Menschen mit ADPKD.

14. Bempedoinsäure zur Verwendung nach Anspruch 13, wobei die wirksame Menge von Bempedoinsäure etwa 30 mg bis etwa 240 mg beträgt.

15. Bempedoinsäure zur Verwendung nach Anspruch 13 oder 14, wobei die Verwendung außerdem die Verabreichung einer wirksamen Menge von Tolvaptan an das Individuum umfasst.

## Revendications

1. Combinaison à dose fixe comprenant de l'acide bempédoïque et du tolvaptan.

2. Combinaison à dose fixe selon la revendication 1, dans laquelle la combinaison à dose fixe comprend environ 30 mg à environ 240 mg d'acide bempédoïque.

3. Combinaison à dose fixe selon la revendication 1 ou 2, dans laquelle la combinaison à dose fixe comprend environ 5 mg à environ 120 mg de tolvaptan.

4. Combinaison à dose fixe selon l'une quelconque des revendications 1 à 3, dans laquelle la combinaison à dose fixe comprend environ 180 mg d'acide bempédoïque.

5. Composition pharmaceutique, comprenant :
de l'acide bempédoïque ;
du tolvaptan ; et
un ou plusieurs excipients pharmaceutiquement acceptables.

6. Composition pharmaceutique selon la revendication 5, dans laquelle la composition pharmaceutique comprend environ 30 mg à environ 240 mg d'acide bempédoïque.

7. Composition pharmaceutique selon la revendication 5 ou 6, dans laquelle la composition pharmaceutique comprend environ 5 mg à environ 120 mg de tolvaptan.

8. Composition pharmaceutique selon l'une quelconque des revendications 5 à 7, dans laquelle la composition pharmaceutique comprend environ 180 mg d'acide bempédoïque.

9. Combinaison à dose fixe selon l'une quelconque des revendications 1 à 4 ou composition pharmaceutique selon l'une quelconque des revendications 5 à 8, dans laquelle la combinaison à dose fixe ou la composition pharmaceutique est formulée pour une administration orale sous la forme d'une forme posologique solide orale.

10. Quantité efficace d'acide bempédoïque pour utilisation dans le traitement de la polykystose rénale autosomique dominante (ADPKD) ou dans le ralentissement de la progression de l'ADPKD chez un humain recevant un traitement par tolvaptan ou dans la prévention de l'insuffisance rénale chez un humain atteint d'ADPKD recevant un traitement par tolvaptan.

11. Acide bempédoïque pour utilisation selon la revendication 10, dans lequel la quantité efficace d'acide bempédoïque est d'environ 30 mg à environ 240 mg.

12. Combinaison à dose fixe selon l'une quelconque des revendications 1 à 4 et 9, ou composition pharmaceutique selon l'une quelconque des revendications 5 à 9, pour utilisation dans le traitement de la polykystose rénale autosomique dominante (ADPKD) ou pour ralentir la progression de l'ADPKD chez un humain qui en a besoin, ou pour prévenir l'insuffisance rénale chez un humain atteint d'ADPKD.

13. Quantité efficace d'acide bempédoïque pour utilisation dans le traitement de la polykystose rénale autosomique dominante (ADPKD) ou pour ralentir la progression de l'ADPKD chez un humain qui en a besoin, ou pour prévenir l'insuffisance rénale chez un humain atteint d'ADPKD.

14. Acide bempédoïque pour utilisation selon la revendication 13, dans lequel la quantité efficace d'acide bempédoïque est d'environ 30 mg à environ 240 mg.

15. Acide bempédoïque pour utilisation selon la revendication 13 ou 14, ladite utilisation comprenant en outre l'administration au sujet d'une quantité efficace de tolvaptan.
